# EUROPEAN PATENT APPLICATION

(11) **EP 2 052 739 A1**
(43) Date of publication of application: **29.04.2009**
(21) Application number: 07792538.6
(22) Date of filing: 15.08.2007
(51) Int. Cl.: A61K 45/00, A61K 38/00, A61P 35/00, A61P 35/04, G01N 33/15, G01N 33/50, G01N 33/53, G01N 33/574

(54) **CANCER THERAPEUTIC AGENT COMPRISING LIGAND FOR NEUROMEDIN U RECEPTOR 2 (FM4) MOLECULE AS ACTIVE INGREDIENT**

(30) Priority: 16.08.2006 JP 2006221866
(71) Applicant: Forerunner Pharma Research Co., Ltd., Meguro-ku Tokyo 153-0041 (JP)
(72) Inventor: KIMURA, Naoki, Tokyo 153-0041 (JP)
(74) Representative: Vossius & Partner
(86) International application number: PCT/JP2007/065904
(87) International publication number: WO 2008/029601

(57) **Abstract**

The present inventors discovered that the neuromedin U receptor 2 (FM4) molecule is highly expressed in cancer cells such as pancreatic cancer cells. When the present inventors measured the proliferation-suppressing effect of ligands of this molecule against cancer cells, the ligands were found to have cancer cell proliferation-suppressing effects. The present inventors also discovered that this effect was produced by an FM4 molecule-mediated signal. These findings showed that ligands for the neuromedin U receptor 2 (FM4) molecule are effective for the treatment of cancers with enhanced expression of the neuromedin U receptor 2 (FM4) molecule, including pancreatic cancer, as well as for prevention of metastasis.

SEQUENCE LISTING

<110> FORERUNNER PHARMA RESEARCH CO., LTD.

<120> CANCER THERAPEUTIC AGENT COMPRISING LIGAND FOR NEUROMEDIN U RECEPTOR 2 (FM4) MOLECULE
AS ACTIVE INGREDIENT

<130> C1-A0614P

<150> JP 2006-221866
<151> 2006-08-16

<160> 20

<170> PatentIn version 3.3

<210> 1
<211> 20
<212> DNA
<213> Artificial

<220>
<223> An artificially synthesized primer sequence

<400> 1
ctcaggcatc caacgcactg 20

<210> 2
<211> 21
<212> DNA
<213> Artificial

<220>
<223> An artificially synthesized primer sequence

<400>  2
ctgaccttct tccattccgt g                                                21

<210>  3
<211>  21
<212>  DNA
<213>  Artificial

<220>
<223>  An artificially synthesized primer sequence

<400>  3
gctatttccg cacgctactg t                                                21

<210>  4
<211>  20
<212>  DNA
<213>  Artificial

<220>
<223>  An artificially synthesized primer sequence

<400>  4
gcccaatgag caggtagagc                                                  20

<210>  5
<211>  20
<212>  DNA
<213>  Artificial

<220>
<223>  An artificially synthesized primer sequence

<400> 5
gggctgctac ttcaagacgg 20

<210> 6
<211> 21
<212> DNA
<213> Artificial

<220>
<223> An artificially synthesized primer sequence

<400> 6
cccttcatct gcctcaagag a 21

<210> 7
<211> 22
<212> DNA
<213> Artificial

<220>
<223> An artificially synthesized primer sequence

<400> 7
atgtcaggga tggaaaaact tc 22

<210> 8
<211> 23
<212> DNA
<213> Artificial

<220>
<223> An artificially synthesized primer sequence

<400> 8
tcaggttttg ttaaagtgga agc 23

<210> 9
<211> 39
<212> DNA
<213> Artificial

<220>
<223> An artificially synthesized primer sequence

<400> 9
aaagaattcc accatgtcag ggatggaaaa acttcagaa 39

<210> 10
<211> 37
<212> DNA
<213> Artificial

<220>
<223> An artificially synthesized primer sequence

<400> 10
tttgcggccg ctcaggtttt gttaaagtgg aagcttt 37

<210> 11
<211> 1248
<212> DNA
<213> Homo sapiens

<400> 11
atgtcaggga tggaaaaact tcagaatgct tcctggatct accagcagaa actagaagat 60

ccattccaga aacacctgaa cagcaccgag gagtatctgg ccttcctctg cggacctcgg 120

cgcagccact tcttcctccc cgtgtctgtg gtgtatgtgc caatttttgt ggtgggggtc 180

attggcaatg tcctggtgtg cctggtgatt ctgcagcacc aggctatgaa gacgcccacc 240

aactactacc tcttcagcct ggcggtctct gacctcctgg tcctgctcct tggaatgccc 300

ctggaggtct atgagatgtg gcgcaactac cctttcttgt tcgggcccgt gggctgctac 360

ttcaagacgg ccctctttga gaccgtgtgc ttcgcctcca tcctcagcat caccaccgtc 420

agcgtggagc gctacgtggc catcctacac ccgttccgcg ccaaactgca gagcacccgg 480

cgccgggccc tcaggatcct cggcatcgtc tggggcttct ccgtgctctt ctccctgccc 540

aacaccagca tccatggcat caagttccac tacttcccca atgggtccct ggtcccaggt 600

tcggccacct ttacggtcat caagcccatg tggatctaca atttcatcat ccaggtcacc 660

tccttcctat tctacctcct ccccatgact gtcatcagtg tcctctacta cctcatggca 720

ctcagactaa agaaagacaa atctcttgag gcagatgaag ggaatgcaaa tattcaaaga 780

ccctgcagaa aatcagtcaa caagatgctg tttgtcttgg tcttagtgtt tgctatctgt 840

tgggccccgt tccacattga ccgactcttc ttcagctttg tggaggagtg gagtgaatcc 900

ctggctgctg tgttcaacct cgtccatgtg gtgtcaggtg tcttcttcta cctgagctca 960

gctgtcaacc ccattatcta taacctactg tctcgccgct tccaggcagc attccagaat 1020

gtgatctctt ctttccacaa acagtggcac tcccagcatg acccacagtt gccacctgcc 1080

cagcggaaca tcttcctgac agaatgccac tttgtggagc tgaccgaaga tataggtccc 1140

caattcccat gtcagtcatc catgcacaac tctcacctcc caacagccct ctctagtgaa 1200

cagatgtcaa gaacaaacta tcaaagcttc cactttaaca aaacctga 1248

<210> 12
<211> 415
<212> PRT
<213> Homo sapiens

<400> 12

Met Ser Gly Met Glu Lys Leu Gln Asn Ala Ser Trp Ile Tyr Gln Gln
1               5                   10                  15

Lys Leu Glu Asp Pro Phe Gln Lys His Leu Asn Ser Thr Glu Glu Tyr
            20                  25                  30

Leu Ala Phe Leu Cys Gly Pro Arg Arg Ser His Phe Phe Leu Pro Val
        35                  40                  45

Ser Val Val Tyr Val Pro Ile Phe Val Val Gly Val Ile Gly Asn Val
    50                  55                  60

Leu Val Cys Leu Val Ile Leu Gln His Gln Ala Met Lys Thr Pro Thr
65                  70                  75                  80

Asn Tyr Tyr Leu Phe Ser Leu Ala Val Ser Asp Leu Leu Val Leu Leu
                85                  90                  95

Leu Gly Met Pro Leu Glu Val Tyr Glu Met Trp Arg Asn Tyr Pro Phe
            100                 105                 110

Leu Phe Gly Pro Val Gly Cys Tyr Phe Lys Thr Ala Leu Phe Glu Thr
        115                 120                 125

Val Cys Phe Ala Ser Ile Leu Ser Ile Thr Thr Val Ser Val Glu Arg
    130                 135                 140

Tyr Val Ala Ile Leu His Pro Phe Arg Ala Lys Leu Gln Ser Thr Arg
145                 150                 155                 160

Arg Arg Ala Leu Arg Ile Leu Gly Ile Val Trp Gly Phe Ser Val Leu
                165                 170                 175

Phe Ser Leu Pro Asn Thr Ser Ile His Gly Ile Lys Phe His Tyr Phe
            180                 185                 190

Pro Asn Gly Ser Leu Val Pro Gly Ser Ala Thr Phe Thr Val Ile Lys
        195                 200                 205

Pro Met Trp Ile Tyr Asn Phe Ile Ile Gln Val Thr Ser Phe Leu Phe
    210                 215                 220

Tyr Leu Leu Pro Met Thr Val Ile Ser Val Leu Tyr Tyr Leu Met Ala
225                 230                 235                 240

Leu Arg Leu Lys Lys Asp Lys Ser Leu Glu Ala Asp Glu Gly Asn Ala
                245                 250                 255
Asn Ile Gln Arg Pro Cys Arg Lys Ser Val Asn Lys Met Leu Phe Val
            260                 265                 270
Leu Val Leu Val Phe Ala Ile Cys Trp Ala Pro Phe His Ile Asp Arg
        275                 280                 285
Leu Phe Phe Ser Phe Val Glu Glu Trp Ser Glu Ser Leu Ala Ala Val
    290                 295                 300
Phe Asn Leu Val His Val Val Ser Gly Val Phe Phe Tyr Leu Ser Ser
305                 310                 315                 320
Ala Val Asn Pro Ile Ile Tyr Asn Leu Leu Ser Arg Arg Phe Gln Ala
                325                 330                 335
Ala Phe Gln Asn Val Ile Ser Ser Phe His Lys Gln Trp His Ser Gln
            340                 345                 350
His Asp Pro Gln Leu Pro Pro Ala Gln Arg Asn Ile Phe Leu Thr Glu
        355                 360                 365
Cys His Phe Val Glu Leu Thr Glu Asp Ile Gly Pro Gln Phe Pro Cys
    370                 375                 380

Gln Ser Ser Met His Asn Ser His Leu Pro Thr Ala Leu Ser Ser Glu
385                 390                 395                 400

Gln Met Ser Arg Thr Asn Tyr Gln Ser Phe His Phe Asn Lys Thr
                405                 410                 415

<210>  13
<211>  75
<212>  DNA
<213>  Homo sapiens

<400>  13
ttcagagtgg acgaagaatt ccaaagtccc tttgcaagtc aaagtcgagg atatttttta      60

ttcaggccac ggaat                                                       75

<210>  14
<211>  25
<212>  PRT
<213>  Homo sapiens

<400>  14

Phe Arg Val Asp Glu Glu Phe Gln Ser Pro Phe Ala Ser Gln Ser Arg
1               5                   10                  15

Gly Tyr Phe Leu Phe Arg Pro Arg Asn
            20                  25

<210>  15
<211>  525

<212>  DNA
<213>  Homo sapiens

<400>  15
atgctgcgaa cagagagctg ccgccccagg tcgcccgccg gacaggtggc cgcggcgtcc      60

ccgctcctgc tgctgctgct gctgctcgcc tggtgcgcgg gcgcctgccg aggtgctcca     120

atattacctc aaggattaca gcctgaacaa cagctacagt tgtggaatga gatagatgat     180

acttgttcgt cttttctgtc cattgattct cagcctcagg catccaacgc actggaggag     240

ctttgcttta tgattatggg aatgctacca aagcctcagg aacaagatga aaaagataat     300

actaaaaggt tcttatttca ttattcgaag acacagaagt tgggcaagtc aaatgttgtg     360

tcgtcagttg tgcatccgtt gctgcagctc gttcctcacc tgcatgagag aagaatgaag     420

agattcagag tggacgaaga attccaaagt ccctttgcaa gtcaaagtcg aggatatttt     480

ttattcaggc cacggaatgg aagaaggtca gcagggttca tttaa                     525


<210>  16
<211>  174
<212>  PRT
<213>  Homo sapiens

<400>  16

Met Leu Arg Thr Glu Ser Cys Arg Pro Arg Ser Pro Ala Gly Gln Val
1               5                   10                  15


Ala Ala Ala Ser Pro Leu Leu Leu Leu Leu Leu Leu Leu Ala Trp Cys
            20                  25                  30

Ala Gly Ala Cys Arg Gly Ala Pro Ile Leu Pro Gln Gly Leu Gln Pro
        35                  40                  45
Glu Gln Gln Leu Gln Leu Trp Asn Glu Ile Asp Asp Thr Cys Ser Ser
    50                  55                  60
Phe Leu Ser Ile Asp Ser Gln Pro Gln Ala Ser Asn Ala Leu Glu Glu
65                  70                  75                  80
Leu Cys Phe Met Ile Met Gly Met Leu Pro Lys Pro Gln Glu Gln Asp
                85                  90                  95
Glu Lys Asp Asn Thr Lys Arg Phe Leu Phe His Tyr Ser Lys Thr Gln
            100                 105                 110
Lys Leu Gly Lys Ser Asn Val Val Ser Ser Val Val His Pro Leu Leu
        115                 120                 125
Gln Leu Val Pro His Leu His Glu Arg Arg Met Lys Arg Phe Arg Val
    130                 135                 140
Asp Glu Glu Phe Gln Ser Pro Phe Ala Ser Gln Ser Arg Gly Tyr Phe
145                 150                 155                 160
Leu Phe Arg Pro Arg Asn Gly Arg Arg Ser Ala Gly Phe Ile
                165                 170

<210> 17
<211> 26
<212> DNA
<213> Artificial

<220>
<223> An artificially synthesized primer sequence

<400> 17
atgctgcgaa cagagagctg ccgccc 26

<210> 18
<211> 26
<212> DNA
<213> Artificial

<220>
<223> An artificially synthesized primer sequence

<400> 18
ttaaatgaac cctgctgacc ttcttc 26

<210> 19
<211> 39
<212> DNA
<213> Artificial

<220>
<223> An artificially synthesized primer sequence

<400> 19
aaagaattcc accatgctgc gaacagagag ctgccgccc 39

<210>  20

<211>  40

<212>  DNA

<213>  Artificial

<220>

<223>  An artificially synthesized primer sequence

<400>  20

tttgcggccg ctcattaaat gaaccctgct gaccttcttc                                        40

## Description

### Technical Field

The present invention relates to methods for diagnosing and treating cancer, and to cell proliferation-suppressing agents and anticancer agents.

### Background Art

Neuromedin U (hereinafter referred to as "NmU") is a biologically active peptide consisting of 23 amino acids, which was isolated in 1985 from the spinal cord of a pig using smooth muscle contraction activity as an index (Non-patent Document 1). Since it has a strong uterotonic activity, the letter "U" from the word "uterus" was used, and it was named "neuromedin U". However, it is not structurally homologous to other biologically active peptides, and its physiological function is hardly elucidated since its discovery 15 years ago.

In 2000, one after another, reports were made by multiple groups suggesting that NmU is an endogenous ligand for orphan GPCRs, FM3 and FM4. Since then, the physiological functions of NmU have been gradually elucidated (Non-patent Documents 2 to 7).

NmU is expressed in the brain and the digestive tract. Specifically in the brain, NmU-producing neurons are present at localized sites such as the hypothalamic arcuate nucleus and the paraventricular nucleus. Administration of NmU into the rat cerebral ventricle showed a significant reduction in overnight food intake and starvation-induced feeding (Non-patent Document 3); and obesity, hyperlipidemia, and fatty liver that accompany overeating, decrease in physical activity, decrease in metabolic activity, and irregular feeding behavior have been observed in NmU-KO mice (Non-patent Document 8). On the other hand, it has been observed that administration of an anti-NmU IgG antibody into the cerebral ventricle led to significant increase in feed intake (Non-patent Document 2). Based on these findings, NmU is considered to function as an endogenous physiologically active peptide that causes suppression of feed intake and enhancement of energy consumption.

First, NmU1R (FM3/GRP66) was cloned as an NmU receptor, and then NmU2R (FM4) was cloned as the second receptor. Although the amino acid sequence homology between FM3 and FM4 is approximately 50%, the expression patterns of the two *in vivo* are largely different. In a living human body, the expression of FM3 has been observed in a wide range of peripheral tissues such as the small intestine, stomach, pancreas, and heart, but not in the brain. In contrast, the expression of FM4 is observed only at localized sites in the brain, and it has not been detected in peripheral tissues other than the testes (Non-patent Document 9).

NmU induced increase of intracellular calcium ion concentration at the nanomolar level in CHO, HEK-293, and COS-7 cells that were forced to express FM3 and FM4. Therefore, NmU is thought to transmit signals into cells via a receptor, and as a result, it may induce some kind of physiological activity in the cells.

Regarding FM3, it is reported that NmU and FM3 are expressed endogenously in K562 cells, which are a human acute myeloid leukemia (AML) cell line. Furthermore, an experiment using K562 has shown that an FM3-mediated autocrine signal caused by NmU induced cell proliferation in K562 cells (Non-patent Document 10).

On the other hand, there are no reports on the expression of FM4 in human-derived cell lines such as cancer cells, and physiological activity induced by FM4-mediated NmU signals has not been analyzed. Similarly, signal analysis using cell lines such as CHO cells and such which are forced to express FM4 has not been carried out. Therefore, the FM4-mediated physiological activity of NmU on cells has not been elucidated at all.

Prior art literature related to the present invention is shown below.
[Non-patent Document 1] N. Minamino, K. Kangawa and H. Matsuo. Neuromedin U-8 and U-25: Novel uterus stimulating and hypertensive peptides identified in porcine spinal cord. Biochem. Biophys. Res. Commun. 130 (1985) 1078-1085.
[Non-patent Document 2] M. Kojima, R. Harunoa, M. Nakazato, Y Date, N. Murakami, R. Hanada, H. Matsuo and K. Kangawa. Purification and Identification of Neuromedin U as an Endogenous Ligand for an Orphan Receptor GPR66 (FM3) Biochem. Biophys. Res. Commun. 276, (2000) 435-438.
[Non-patent Document 3] Howard AD, Wang RP, Pong SS, Mellin TN, Strack A, Guan XM, Zeng ZZ, Williams DL, Feighner SD, Nunes CN, et al. Identification of receptors for neuromedin U and its role in feeding. Nature 406 (2000) 70-74.
[Non-patent Document 4] Szekeres PG, Muir AI, Spinage LD, Miller JE, Butler SI, Smith A, Rennie GI, Murdock PR, Fitzgerald LR, Wu HL, et al. Neuromedin U is a potent agonist at the orphan G protein-coupled receptor FM3. J Biol Chem 275 (2000) 20247-20250.
[Non-patent Document 5] R. Fujii, M. Hosoya, S. Fukusumi, Y Kawamata, Y Habata, S. Hinuma, H. Onda, O. Nishimura and M. Fujino. Identification of neuromedin U as the cognate ligand of the orphan G protein-coupled receptor FM-3. J. Biol. Chem. 275 (2000) 21068-21074.
[Non-patent Document 6] Hosoya M, Moriya T, Kawamata Y, Ohkubo S, Fujii R, Matsui H, Shintani Y, Fukusumi S, Habata Y, Hinuma S, et al. Identification and functional characterization of a novel subtype of neuromedin U receptor. J Biol Chem 275 (2000) 29528-29532.
[Non-patent Document 7] Raddatz R, Wilson AE, Artymyshyn R, Bonini JA, Borowsky B, Boteju LW, Zhou SQ, Kouranova EV, Nagomy R, Guevarra MS, et al. Identification and characterization of two neuromedin U receptors differentially expressed in peripheral tissues and the central nervous system. J Biol Chem 275 (2000) 32452-32459.
[Non-patent Document 8] Hanada R, Teranishi H, Pearson JT, Kurokawa M, Hosoda H, Fukushima N, Fukue Y, Serino R, Fujihara H, Ueta Y, Ikawa M, Okabe M, Murakami N, Shirai M, Yoshimatsu H, Kangawa K, Kojima M. Neuromedin U has a novel anorexigenic effect independent of the leptin signaling pathway. Nat Med. 10 (2004) 1067-73.
[Non-patent Document 9] Paul J. Brighton, Philip G. Szekeres and Gary B. Willars. Neuromedin U and Its Receptors: Structure, Function, and Physiological Roles. Pharmacol Rev 56 (2004) 231-248.
[Non-patent Document 10] Shetzline SE, Rallapalli R, Dowd KJ, Zou S, Nakata Y, Swider CR, Kalota A, Choi JK, Gewirtz AM. Neuromedin U: A Myb-regulated autocrine growth factor for human myeloid leukemias. Blood. 104 (2004) 1833-40.

### Disclosure of the Invention

### [Problems to be Solved by the Invention]

An objective of the present invention is to provide ligands for the neuromedin U receptor 2 (FM4) molecule and uses thereof. More specifically, an objective is to provide novel methods for treating cancer using ligands for the neuromedin U receptor 2 (FM4) molecule, and novel cell proliferation-suppressing agents and anti-cancer agents comprising a ligand for the neuromedin U receptor 2 (FM4) molecule.

### [Means for Solving the Problems]

The present inventors discovered that the neuromedin U receptor 2 (FM4) molecule is highly expressed in cancer cells such as pancreatic cancer cells. Furthermore, when the present inventors measured the proliferation-suppressing effect of a ligand of the neuromedin U receptor 2 (FM4) molecule on cancer cells such as pancreatic cancer cells, they discovered that this ligand has an effect of suppressing cancer cell proliferation and that this effect is produced by FM4 molecule-mediated signals. Furthermore, when the present inventors evaluated the colony formation-suppressing effect of a ligand of the neuromedin U receptor 2 (FM4) molecule on cancer cells such as pancreatic cancer cells, they discovered that this ligand has an effect of suppressing colony formation, and that this effect is induced through the FM4 molecule. In addition, when the present inventors evaluated the cell movement-suppressing effect of a ligand of the neuromedin U receptor 2 (FM4) molecule on cancer cells such as pancreatic cancer cells, they discovered that this ligand has an effect of suppressing cell movement, and that this effect is induced through the FM4 molecule. From the above-mentioned findings, the present inventors discovered that ligands of the neuromedin U receptor 2 (FM4) molecule are effective for cancer therapy and metastasis prevention against neuromedin U receptor 2 (FM4) molecule-overexpressing cancers, including pancreatic cancer, and thereby completed the present invention.

Thus, the present invention provides pharmaceutical compositions that comprise a ligand of the neuromedin U receptor 2 (FM4) molecule as an active ingredient. More specifically, the present invention provides cell proliferation-suppressing agents comprising a ligand of the neuromedin U receptor 2 (FM4) molecule as an active ingredient. Furthermore, the present invention provides colony formation-suppressing agents comprising a ligand of the neuromedin U receptor 2 (FM4) molecule as an active ingredient. The present invention also provides cell movement-suppressing agents comprising a ligand of the neuromedin U receptor 2 (FM4) molecule as an active ingredient. The present invention further provides cancer therapeutic agents comprising a ligand of the neuromedin U receptor 2 (FM4) molecule as an active ingredient. In addition, the present invention provides cancer metastasis-suppressing agents comprising a ligand of the neuromedin U receptor 2 (FM4) molecule as an active ingredient.

A ligand of the neuromedin U receptor 2 (FM4) molecule in the above-mentioned pharmaceutical agents of the present invention is preferably a naturally-occurring or artificial ligand, and more preferably, a naturally-occurring ligand. Particularly preferred naturally-occurring ligands are, for example, human-derived ligands, and more preferred ligands are, for example, neuromedin U and peptides that have a substantially identical agonist activity as neuromedin U. Such peptides include, for example, a polypeptide comprising the amino acid sequence of GenBank No. P48645 (SEQ ID NO: 14) and peptides that are substantially identical to a polypeptide comprising this amino acid sequence. The type of cancer that is particularly preferred as a target of the anticancer agent is, for example, pancreatic cancer.

In another embodiment, the present invention provides use of the neuromedin U receptor 2 (FM4) molecule as a diagnostic marker for cancer.

In another embodiment, the present invention provides methods for suppressing the proliferation of cells that express the neuromedin U receptor 2 (FM4) molecule by contacting the cells that express the neuromedin U receptor 2 (FM4) molecule with a ligand of the neuromedin U receptor 2 (FM4) molecule. Furthermore, in another embodiment, the present invention provides methods for suppressing colony formation of cells that express the neuromedin U receptor 2 (FM4) molecule by contacting the cells that express the neuromedin U receptor 2 (FM4) molecule with a ligand of the neuromedin U receptor 2 (FM4) molecule. In addition, in another embodiment, the present invention provides methods for suppressing movement of cells that express the neuromedin U receptor 2 (FM4) molecule by contacting cells that express the neuromedin U receptor 2 (FM4) molecule with a ligand of the neuromedin U receptor 2 (FM4) molecule.

In the above-mentioned methods of the present invention, a ligand of the neuromedin U receptor 2 (FM4) molecule is preferably a naturally-occurring or artificial ligand. The cells that express the neuromedin U receptor 2 (FM4) molecule are preferably cancer cells, and more preferably pancreatic cancer cells.

Furthermore, in another embodiment, the present invention provides methods of screening for ligands for the neuromedin U receptor 2 (FM4) protein, which use the neuromedin U receptor 2 (FM4) protein-mediated cell-stimulating activity as an index. More specifically, the present invention provides:
[1] a cancer therapeutic agent comprising as an active ingredient a ligand for a polypeptide comprising the amino acid sequence of SEQ ID NO: 12 or a polypeptide functionally equivalent thereto;
[2] the cancer therapeutic agent of [1], wherein the ligand is a polypeptide comprising the amino acid sequence of SEQ ID NO: 14 or a polypeptide functionally equivalent thereto;
[3] the cancer therapeutic agent of [1] or [2], wherein the cancer is pancreatic cancer;
[4] a cancer metastasis-suppressing agent comprising as an active ingredient a ligand for a polypeptide comprising the amino acid sequence of SEQ ID NO: 12 or a polypeptide functionally equivalent thereto;
[5] the cancer metastasis-suppressing agent of [4], wherein the ligand is a polypeptide comprising the amino acid sequence of SEQ ID NO: 14 or a polypeptide functionally equivalent thereto;
[6] the cancer metastasis-suppressing agent of [4] or [5], wherein the cancer is pancreatic cancer;
[7] a cell proliferation-suppressing agent comprising as an active ingredient a ligand for a polypeptide comprising the amino acid sequence of SEQ ID NO: 12 or a polypeptide functionally equivalent thereto;
[8] the cell proliferation-suppressing agent of [7], wherein the ligand is a polypeptide comprising the amino acid sequence of SEQ ID NO: 14 or a polypeptide functionally equivalent thereto;
[9] the cell proliferation-suppressing agent of [7] or [8], wherein the cells are pancreatic cancer cells;
[10] a method of screening for a ligand for a polypeptide comprising the amino acid sequence of SEQ ID NO: 12 or a polypeptide functionally equivalent thereto, which comprises the steps of:
   (a) contacting a test substance with a cell expressing a polypeptide comprising the amino acid sequence of SEQ ID NO: 12 or a polypeptide functionally equivalent thereto, or with an extract of said cell;
   (b) measuring cell-stimulating activity of the test substance in the cell of step (a) or the extract of said cell; and
   (c) selecting a test substance that alters the above-mentioned cell-stimulating activity as compared to when the test substance is not contacted;
[11] a method of screening for a ligand for a polypeptide comprising the amino acid sequence of SEQ ID NO: 12 or a polypeptide functionally equivalent thereto, which comprises the steps of:
   (a) contacting a test substance with a cell expressing a polypeptide comprising the amino acid sequence of SEQ ID NO: 12 or a polypeptide functionally equivalent thereto, or with an extract of said cell;
   (b) measuring cell-stimulating activity of the test substance in the cell of step (a) or the extract of said cell; and
   (c) selecting a test substance that alters the above-mentioned cell-stimulating activity as compared to when neuromedin U is contacted;
[12] the method of [10] or [11], wherein the cell is a recombinant cell;
[13] the method of [12], wherein the recombinant cell is a cell derived from CHO or PANC1;
[14] the method of any one of [10] to [13], wherein the cell-stimulating activity is intracellular Ca²⁺ concentration-increasing activity;
[15] the method of any one of [10] to [13], wherein the cell-stimulating activity is cell proliferation-suppressing activity;
[16] the method of any one of [10] to [13], wherein the cell-stimulating activity is activity of suppressing cell colony formation;
[17] the method of any one of [10] to [13], wherein the cell-stimulating activity is activity of suppressing cell movement;
[18] a ligand obtained by the method of any one of [10] to [17];
[19] a method for diagnosing a cancer, which comprises the step of detecting the expression level of a polynucleotide encoding a polypeptide comprising the amino acid sequence of SEQ ID NO: 12 or a polypeptide functionally equivalent thereto in a biological sample of a specimen,
   wherein the specimen is diagnosed with cancer when the expression level of a polynucleotide encoding the polypeptide comprising the amino acid sequence of SEQ ID NO: 12 or the polypeptide functionally equivalent thereto is increased as compared to a normal tissue;
[20] the method of [19], wherein the detection is performed using as a probe a polynucleotide encoding a polypeptide comprising the amino acid sequence of SEQ ID NO: 14 or a polypeptide functionally equivalent thereto, or a fragment thereof;
[21] the method of [20], wherein the polynucleotide comprises the nucleotide sequence of SEQ ID NO: 13;
[22] the method of any one of [19] to [21], wherein the cancer is pancreatic cancer;
[23] a method for treating cancer, which comprises the step of administering to a subject a ligand for a polypeptide comprising the amino acid sequence of SEQ ID NO: 12 or a polypeptide functionally equivalent thereto;
[24] a method for suppressing cancer metastasis, which comprises the step of administering to a subject a ligand for a polypeptide comprising the amino acid sequence of SEQ ID NO: 12 or a polypeptide functionally equivalent thereto;
[25] a method for suppressing cell proliferation, which comprises the step of administering to a subject a ligand for a polypeptide comprising the amino acid sequence of SEQ ID NO: 12 or a polypeptide functionally equivalent thereto.
[26] use of a ligand for a polypeptide comprising the amino acid sequence of SEQ ID NO: 12 or a polypeptide functionally equivalent thereto in the manufacture of a cancer therapeutic agent;
[27] use of a ligand for a polypeptide comprising the amino acid sequence of SEQ ID NO: 12 or a polypeptide functionally equivalent thereto in the manufacture of a cancer metastasis-suppressing agent; and
[28] use of a ligand for a polypeptide comprising the amino acid sequence of SEQ ID NO: 12 or a polypeptide functionally equivalent thereto in the manufacture of a cell proliferation-suppressing agent.

### Brief Description of the Drawings

Fig. 1a depicts results of the GeneChip analysis. Fig. 1a shows comparison of the NmU expression in human tissues.
Fig. 1b depicts results of the GeneChip analysis. Fig. 1b shows comparison of the NmU expression in human-derived cancer cell lines.
Fig. 2 shows results of the expression analysis of NmU and its receptor gene NmU-R by RT-PCR. Fig. 2(A) depicts the comparison of expression of the NmU gene and the g3pdh (internal control) gene in pancreatic cancer cells and normal tissues. Fig. 2(B) depicts the comparison of expression of two types ofNmU-Rs, FM3 and FM4, in pancreatic cancer cells and normal tissues.
Fig. 3 depicts the result of analyzing the responsiveness of CHO cells and FM4-expressing CHO cells (FM4-CHO) to Nmu stimulation by using the change in intracellular Ca²⁺ concentration.
Fig. 4 shows the effect of NmU on cell proliferation. Fig 4(A) depicts the effect of NmU on cell proliferation in FM4-expressing CHO cells (FM4-CHO). Fig. 4(B) depicts the effect of NmU on cell proliferation in two types of pancreatic cancer cells, PANC-1 and CAPAN-1.
Fig. 5 depicts the result of analyzing the expression of the FM4 gene in FM4-expressing PANC1 cells (FM4-PANC1) by RT-PCR.
Fig. 6 shows the result of analyzing the responsiveness to NmU in NmU-stimulated PANC1 cells and FM4-expressing PANC1 cells (FM4-PANC1), by using the change in intracellular Ca²⁺ concentration.
Fig. 7 shows the result of analyzing the suppressive effect of NmU on colony formation of FM4-expressing PANC1 cells (FM4-PANC1). Fig. 7(A) shows the colonies. Fig. 7(B) is a graph showing the change in the number of colonies as a result of NmU addition.
Fig. 8 shows changes in the morphology of FM4-expressing CHO cells (FM4-CHO).
Fig. 9 shows the effect of NmU on cell movement. Fig. 9(A) shows the effect of NmU stimulation on CHO cell movement. Fig. 9(B) shows the effect of NmU stimulation on the cell movement of FM4-expressing CHO cells (FM4-CHO).

### Best Mode for Carrying Out the Invention

### 1. Neuromedin U receptor 2 (FM4) and genes encoding this receptor

The amino acid sequence of the naturally-occurring neuromedin U receptor 2 (FM4), and its encoding gene sequence are disclosed in GenBank Nos. NP_064552 (SEQ ID NO: 12) and NM_020167 (SEQ ID NO: 11), respectively. In the present invention, neuromedin U receptor 2 (FM4) (hereinafter, it may be referred to as the protein of the present invention) refers to a polypeptide comprising the amino acid sequence of SEQ ID NO: 12, or a functionally equivalent polypeptide. A polypeptide functionally equivalent to a polypeptide comprising the amino acid sequence of SEQ ID NO: 12 is, for example, a fragment of the polypeptide comprising the amino acid sequence of SEQ ID NO: 12. A fragment of neuromedin U receptor 2 (FM4) refers to a polypeptide that comprises any region of the naturally-occurring neuromedin U receptor 2 (FM4) protein and has substantially equivalent function or activity as the naturally-occurring neuromedin U receptor 2 (FM4) protein. Substantially equivalent function or activity as the naturally-occurring neuromedin U receptor 2 (FM4) protein is, for example, ligand binding activity or signal transduction effect.

The functionally equivalent polypeptides of neuromedin U receptor 2 (FM4) in the present invention may also include a polypeptide that has biological activity equivalent to that of the polypeptide comprising the amino acid sequence of SEQ ID NO: 12. Thus, a more specific embodiment of the functionally equivalent polypeptides of neuromedin U receptor 2 (FM4) in the present invention is, for example, a protein comprising an amino acid sequence with one or more amino acid substitutions, deletions, insertions, and/or additions in the amino acid sequence of SEQ ID NO: 12, or a protein encoded by a nucleic acid that hybridizes under stringent conditions with a nucleic acid comprising the nucleotide sequence of SEQ ID NO: 11, and which is functionally equivalent to a protein comprising the amino acid sequence of SEQ ID NO: 12.

Methods well known to those skilled in the art for preparing proteins that are functionally equivalent to a certain protein include the method of introducing mutations into the protein. For example, those skilled in the art can prepare mutants that are functionally equivalent to neuromedin U receptor 2 (FM4) by introducing appropriate mutations into the amino acids of neuromedin U receptor 2 (FM4) using site-directed mutagenesis (Hashimoto-Gotoh, T, Mizuno, T, Ogasahara, Y, and Nakagawa, M. (1995) An oligodeoxyribonucleotide-directed dual amber method for site-directed mutagenesis. Gene 152, 271-275; Zoller, MJ, and Smith, M.(1983) Oligonucleotide-directed mutagenesis of DNA fragments cloned into M13 vectors. Methods Enzymol. 100, 468-500; Kramer, W., Drutsa, V., Jansen, H.W., Kramer, B., Pflugfelder, M., and Fritz, H.J. (1984) The gapped duplex DNA approach to oligonucleotide-directed mutation construction. Nucleic Acids Res. 12, 9441-9456; Kramer W, and Fritz HJ (1987) Oligonucleotide-directed construction of mutations via gapped duplex DNA Methods. Enzymol. 154, 350-367; Kunkel, TA (1985) Rapid and efficient site-specific mutagenesis without phenotypic selection. Proc. Natl. Acad. Sci. U S A. 82, 488-492) or the like. Amino acid mutations in proteins may also occur in nature. Therefore, a protein having an amino acid sequence with one or more amino acid mutations in the amino acid sequence of neuromedin U receptor 2 (FM4) (SEQ ID NO: 12), and which is functionally equivalent to this protein is also included in the proteins of the present invention.

When modifying amino acid residues, it is desirable to mutate them into amino acids in which the properties of the amino acid side chains are conserved. Examples of amino acid side chain properties include: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G. A, V, L, I, and P); hydroxyl group-containing side chains (S, T, and Y); sulfur atom-containing side chains (C and M); carboxylic acid- and amide-containing side chains (D, N, E, and Q); basic side chains (R, K, and H); and aromatic ring-containing side chains (H, F, Y, and W) (amino acids are represented by one-letter codes in parentheses). Amino acid substitutions within each of these groups are referred to as conservative substitutions. Polypeptides comprising a modified amino acid sequence, in which one or more (preferably 1 to 10 or so, and more preferably 1 to 5 or so) amino acid residues in a certain amino acid sequence is deleted, added, and/or substituted with other amino acids, are known to retain their biological activities (Mark, D. F. et al., Proc. Natl. Acad. Sci. USA (1984) 81: 5662-5666; Zoller, M. J. & Smith, M. Nucleic Acids Research (1982) 10: 6487-500; Wang, A. et al., Science (1984) 224: 1431-3; Dalbadie-McFarland, G. et al., Proc. Natl. Acad. Sci. USA (1982) 79: 6409-6413). Such mutants have amino acid sequence identity of at least 70%, preferably at least 75%, more preferably at least 80%, even more preferably at least 85%, still more preferably at least 90%, and most preferably at least 95% to the amino acid sequence of the protein of the present invention. Herein, sequence identity is defined as the percentage of residues that are identical to the residues of the amino acid sequence of the original protein after the necessary alignment and insertion of suitable gaps to maximize the sequence identity. Amino acid sequence identity can be determined by the above-described method. Nucleotide sequence identity and amino acid sequence identity can be determined, for example, by the later-described BLAST algorithm by Karlin and Altschul (Proc. Natl. Acad. Sci. USA (1993) 90: 5873-7).

Meanwhile, a DNA encoding neuromedin U receptor 2 (FM4) (hereinafter, it may be referred to as the DNA of the present invention) is, for example, the aforementioned DNA encoding neuromedin U receptor 2 (FM4) or a DNA functionally equivalent thereto. A specific embodiment of a DNA encoding neuromedin U receptor 2 (FM4) is, for example, the DNA of SEQ ID NO: 11. A DNA functionally equivalent to the DNA encoding neuromedin U receptor 2 (FM4) is, for example, a DNA comprising a nucleotide sequence encoding a fragment of neuromedin U receptor 2 (FM4) and having functions or activities (for example, ligand binding activity or signal transduction effect) that are substantially identical to those of neuromedin U receptor 2 (FM4) when expressed in suitable cells. A specific embodiment of such DNA is, for example, a fragment of the DNA of SEQ ID NO: 11.

An example of a DNA functionally equivalent to the neuromedin U receptor 2 (FM4)-encoding DNA of the present invention may also be a polynucleotide having biological activity equivalent to that of a DNA comprising the nucleotide sequence of SEQ ID NO: 11. Thus, a more specific embodiment of a DNA functionally equivalent to the neuromedin U receptor 2 (FM4)-encoding DNA in the present invention is, for example, a DNA comprising a nucleotide sequence with one or more nucleotide substitutions, deletions, insertions, and/or additions in the nucleotide sequence of SEQ ID NO: 11, or a DNA that hybridizes under stringent conditions with a DNA consisting of the nucleotide sequence of SEQ ID NO: 11, and which encodes a protein that is functionally equivalent to a protein consisting of the amino acid sequence of SEQ ID NO: 11.

The DNA of the present invention may be any one of genomic DNA, genomic DNA library, the aforementioned cell- or tissue-derived cDNA, the aforementioned cell- or tissue-derived cDNA library, or synthetic DNA, as long as it meets the above-mentioned conditions. A vector to be used for expression in suitable cells may be any one of bacteriophage, plasmid, cosmid, and phagemid.

The protein and DNA of the present invention are useful, for example, when performing a receptor binding assay using a neuromedin U receptor 2 (FM4) expression system. A receptor binding assay system that uses a neuromedin U receptor 2 (FM4) expression system can be use to screen for human- or mammal-specific ligands for neuromedin U receptor 2 (FM4). Ligands obtained from this screening can be used as preventive/therapeutic agents or such for various types of diseases.

The neuromedin U receptor 2 (FM4)-encoding DNA and DNAs functionally equivalent thereto can be directly amplified by reverse transcription polymerase chain reaction (hereinafter referred to as RT-PCR) using synthetic DNA primers containing a partial nucleotide sequence of the nucleotide sequence of a DNA encoding the protein of the present invention, and prepared RNA fractions containing mRNAs from cells/tissues expressing neuromedin U receptor 2 (FM4) as template.

Specifically, for example, a DNA comprising the nucleotide sequence of SEQ ID NO: 11 is used as a neuromedin receptor 2 (FM4)-encoding DNA or a DNA functionally equivalent thereto. Furthermore, even if a DNA has one or more nucleotide additions, deletions, or substitutions in any of the nucleotides of this nucleotide sequence, it can be suitably used if it has a function or activity (for example, ligand binding activity or signal transduction effect) that is substantially identical to that of neuromedin U receptor 2 (FM4) when expressed in suitable cells. For the synthetic DNA primer, a DNA comprising the nucleotide sequence of any one of SEQ ID NOs: 7, 8, 9, and 10 can be suitably used, but it is not limited thereto. That is, any sequence can be suitably used if it can amplify a neuromedin U receptor 2 (FM4)-encoding DNA that has a function or activity (for example, ligand binding activity or signal transduction effect) substantially identical to that of neuromedin U receptor 2 (FM4) when expressed in suitable cells. Synthetic DNA primers having specific sequences can be prepared by a solid phase synthesis method using a semi-automated synthesizer (for example, Models 392/394 or such manufactured by PE Applied Biosystems).

When amplifying a DNA that fully encodes neuromedin U receptor 2 (FM4) by the PCR method, an RNA library containing various types of mRNAs obtained from neuromedin U receptor 2 (FM4)-expressing cells can be suitably used as template. The neuromedin U receptor 2 (FM4)-expressing cells are not particularly limited, and cancer cell lines related to the present invention, or preferably Capan-1, Capan-2, CFPAC1, HPAFII, AsPC, MPANC96, su.86.86, or such which are pancreatic cancer cell lines, can be suitably used in addition to cells derived from the tissues of substantia nigra, medulla oblongata, pontine reticular formation, spinal cord, thalamus, hippocampus, hypothalamus, cerebral cortex, and testis. For RNA isolation, total RNA can be prepared by known methods, for example, the guanidine ultracentrifugation method (Chirgwin, J. M. et al., Biochemistry 18, 5294-5299, (1979)), or the AGPC method (Chomczynski, P. and Sacchi, N., Anal. Biochem. 162, 156-159, (1987)). mRNAs can be purified from total RNA prepared in this manner by using an mRNA purification kit (Pharmacia), or the like. Furthermore, by using the QuickPrep mRNA Purification Kit (Pharmacia), mRNAs can be prepared directly from the above-described neuromedin U receptor 2 (FM4)-expressing cells.

In addition to the above-described RNA library, a cDNA library produced from this RNA library can also be suitably used. A cDNA library is synthesized from the obtained mRNAs using reverse transcriptase. It is possible to synthesize a cDNA library using the AMV Reverse Transcriptase First-strand cDNA Synthesis Kit (Seikagaku Corporation) or such. Furthermore, a cDNA library can be synthesized and amplified according to the 5'-RACE method (Frohman, M. A. et al., Proc. Natl. Acad. Sci. U.S.A. (1988) 85, 8998-9002, (1988); Belyavsky, A. et al., Nucleic Acids Res. 17, 2919-2932, (1989)) by using the 5'-Ampli FINDER RACE Kit (Clontech) and polymerase chain reaction (hereinafter referred to as PCR). Such a cDNA library can be obtained from commercially available cDNA libraries.

cDNA clones containing a neuromedin U receptor 2 (FM4)-encoding cDNA can be selected by hybridization from a cDNA library produced by incorporation in a suitable vector. When performing this hybridization, it is possible to use, other than the oligonucleotide of SEQ ID NO: 5 or 6, for example, an oligonucleotide that has a continuous sequence within the polynucleotide sequence of SEQ ID NO: 11 or a nucleotide sequence complementary thereto, and is of at least 15 nucleotides, preferably at least 30 nucleotides, more preferably at least 50 nucleotides, and at most 2000 nucleotides, preferably at most 1000 nucleotides, and more preferably at most 500 nucleotides. Alternatively, the polynucleotide sequence of SEQ ID NO: 11, or a nucleotide sequence complementary thereto is suitably used. The above-described methods can be suitably used for synthesizing these DNAs. The method of cDNA library production, probe labeling method, reaction conditions for hybridization, and methods used for hybridization can be performed according to methods described in Molecular Cloning 2nd Ed. (J. Sambrook et al., Cold Spring Harbor Lab. Press, 1989). When using a commercially available library, they can be carried out according to the method described in the attached instructions of use.

The term "hybridization" means that a DNA or a corresponding RNA binds by hydrogen bonding interaction to another DNA or RNA molecule in solution or on a solid support. The strength of such an interaction can be evaluated by changing the stringency of hybridization conditions. Hybridization conditions of various stringencies may be used depending on the desired specificity and selectivity. The stringency can be adjusted by changing the salt concentration or the concentration of a denaturing agent. Methods for adjusting the stringency are described in Molecular Cloning mentioned above and well known in the art.

"Stringent hybridization conditions" refers to conditions in the presence of 50% formamide, at 42°C in 700 mM NaCl, or equivalent conditions. One example of the stringent hybridization conditions is overnight hybridization at 42°C in a solution of 50% formamide, 5x SSC, 50 mM NaH₂PO₄, pH6.8, 0.5% SDS, 0.1 mg/mL sonicated salmon sperm DNA, and 5x Denhardt's solution; washing at 45°C with 2x SSC and 0.1% SDS; and washing at 45°C with 0.2x SSC and 0.1% SDS.

### 2. Methods and compositions for diagnosing cancer

According to the present invention, cancer cells including pancreatic cancer cells can be diagnosed using a neuromedin U receptor 2 (FM4) molecule-encoding DNA obtained based on the above description. More specifically, the present invention provides methods for diagnosing the presence of cancer cells in a test sample using test samples such as tissue fragments, blood, cells, or the like obtained by biopsy or such from test patients. Furthermore, the present invention provides compositions for detecting the presence of cancer cells, that is, compositions for diagnosing cancer. Specifically, a composition for diagnosis of the present invention may include a set of primers that can amplify an oligonucleotide comprising the nucleotide sequence of SEQ ID NO: 11 (neuromedin U receptor 2 (FM4)-encoding DNA). Furthermore, the present invention provides primers that can amplify oligonucleotides comprising the nucleotide sequence of SEQ ID NO: 11 (DNA encoding neuromedin U receptor 2 (FM4)). Such primers are, for example, polynucleotides comprising the nucleotide sequence of SEQ ID NO: 15. By performing polymerase chain reaction (PCR) using these primers and RNAs extracted from a test sample by the above-described method or cDNAs prepared from these RNAs as template, the sequence of interest can be amplified. Furthermore, the presence or quantitative change of neuromedin U receptor 2 (FM4) transcript in the test sample can be detected. Such PCR method is well known in the technical field and is described, for example, in "PCR Protocols, A Guide to Methods and Applications", Academic Press, Michael, et al., eds. 1990.

A more quantitative method for measuring the amount or change of the amount of neuromedin U receptor 2 (FM4) transcript present in a test sample, for example, quantitative RT-PCR. A quantitative RT-PCR method is a method that detects and analyzes the production process of PCR amplification products in real time using an instrument that integrates a spectrophotofluorometer and a thermal cycler used for PCR reactions. To perform quantitative PCR using an internal standard, it is necessary to compare the amount of amplified product with one that has the same amplification efficiency during exponential amplification. Since the process of producing amplification products can be observed continuously in quantitative RT-PCR, more accurate quantification is possible. Quantitative PCR kits and systems comprising a measuring instrument are commercially available (for example, "iCycler iQ Real-Time PCR System" from Bio-Rad), and they can be used by following the manual attached to such systems or kits. A DNA encoding g3pdh (glyceraldehyde dehydrogenase) or ACT (actin) is suitable for use as internal standard.

To be suitable for use as primers, the oligonucleotides of the present invention should have a continuous sequence of at least 12 or more nucleotides, preferably 12 to 50 nucleotides, and more preferably 12 to 20 nucleotides within the nucleotide sequence of SEQ ID NO: 11 or a nucleotide sequence complementary thereto. A more preferred example is the nucleotides of SEQ ID NO: 5 or 6.

Furthermore, *in situ* hybridization, which is a method for visually detecting DNAs and/or RNAs expressed in cells, can also be suitably used. By reacting a labeled probe with a tissue section, cell sample, or the like, expression of the neuromedin U receptor 2 (FM4) molecule in a localized region of a tissue can be made visible in tissue section or cell sample.

To be suitable for use as a probe in *in situ* hybridization methods, the oligonucleotides of the present invention should have a continuous sequence of at least 15 nucleotides, preferably at least 30 nucleotides, more preferably at least 50 nucleotides, but at the same time, at most 1000 nucleotides, preferably at most 500 nucleotides, and more preferably at most 300 nucleotides within the nucleotide sequence of SEQ ID NO: 11, or a nucleotide sequence complementary thereto. Such a probe can be labeled using a hot method which is a method of labeling with radioactive phosphorus.

One can also suitably use an *in situ* PCR method in which a DNA encoding the neuromedin U receptor 2 (FM4) molecule is amplified by PCR in advance, and then detected by the *in situ* hybridization method. Primers used in the *in situ* PCR method which are specific to the sequence of the neuromedin U receptor 2 (FM4) molecule are, for example, the above-described oligonucleotides, or more specifically, oligonucleotides having a continuous sequence of at least 12 nucleotides or more, preferably 12 to 50 nucleotides, or more preferably 12 to 20 nucleotides within the nucleotide sequence of SEQ ID NO: 11 or a nucleotide sequence complementary thereto. A specific example is the oligonucleotide of SEQ ID NO: 5 or 6. The technique of the *in situ* PCR method is well known in this technical field, and is described, for example, in "Cell and Molecular Biology, In-Situ PCR Techniques", Wiley, Omar Bagasra & John Hansen, eds. 1997. The cancers in the diagnostic method of the present invention are not particularly limited, and may be any cancer such as lung cancer, colon cancer, pancreatic cancer, and stomach cancer. A preferred example of the cancer is pancreatic cancer.

### 3. Recombinant cells that express neuromedin U receptor 2 (FM4)

The present invention further provides recombinant cells that express neuromedin U receptor 2 (FM4). A DNA that encodes neuromedin U receptor 2 (FM4) may have ATG at its 5' end as translation initiation codon and a TAA, TGA, or TAG at its 3' end as translation termination codon. These translation initiation and translation termination codons may be added by using appropriate synthetic DNA adapters. Expression vectors for neuromedin U receptor 2 (FM4), can be produced by, for example, (a) excising a DNA fragment of interest from DNAs comprising a neuromedin U receptor 2 (FM4)-encoding DNA, and (b) ligating the DNA fragment downstream of a promoter in a suitable expression vector.

Examples of vectors that are used include plasmids derived from *Escherichia coli* (for example, pBR322, pBR325, and pUC19); plasmids derived from *Bacillus subtilis* (for example, pUB110, pC 194, and pE194Ts); plasmids derived from yeast (for example, pSH19); bacteriophages such as λ-phage; animal viruses such as retrovirus, vaccinia virus, and baculovirus; as well as pA1-11, pXT1, pRc/CMV, pRC/RSV, pcDNAI/Neo, pcDNA3.1, pRC/CMV2, pRc/RSV (Invitrogen), and so on. Promoters used in the present invention may be any promoter as long as it is appropriate for the host used for gene expression. When animal cells are used as host, examples include the SRα promoter, SV40 promoter, HIV-LTR promoter, CMV promoter, and HSV-TK promoter. Of these, the CMV promoter, SRα promoter, or the like is preferably used. When the host is a bacterium of the genus *Escherichia,* the trp promoter, lac promoter, recA promoter, λPL promoter, lpp promoter, or the like is preferred; when the host is a bacterium of the genus *Bacillus,* the SPO1 promoter, SPO2 promoter, or the like is preferred; and when the host is yeast, the PHO5 promoter, PGK promoter, GAP promoter, ADH promoter, or the like is preferred. When the host is an insect cell, the polyhedrin promoter, P10 promoter, or the like is preferred.

Other than the above, one may use as expression vectors, those comprising, if desired, enhancers, splicing signals, polyadenylation signals, selection markers, SV40 replication origin (hereinafter, sometimes referred to as "SV40 ori") and the like. Examples of selection markers include the dihydrofolate reductase (hereinafter, sometimes referred to as "dhfr") gene, methotrexate (MTX) resistance gene, ampicillin resistance gene (hereinafter, sometimes referred to as "Ampr"), neomycin resistance gene (hereinafter, sometimes referred to as "Neor"; G418 resistance), and the like. In particular, when CHO(dhfr-) cells are used with the dhfr gene as a selection marker, the gene of interest can be selected using a thymidine-free medium. Furthermore, when necessary, a signal sequence appropriate for the host may be added to the N terminus of the protein of the present invention. When the host is a bacterium of the genus *Escherichia*, a PhoA signal sequence, OmpA signal sequence, or the like may be used; when the host is a bacterium of the genus *Bacillus,* an α-amylase signal sequence, subtilisin signal sequence, or the like may be used; when the host is yeast, an MFα signal sequence, SUC2 signal sequence, or the like may be used; and when the host is an animal cell, an insulin signal sequence, α-interferon signal sequence, or the like may be used. Transformants can be produced by using the thus constructed vector comprising a DNA encoding the neuromedin U receptor 2 (FM4) protein.

Examples of hosts that may be used include bacteria of the genus *Escherichia*, bacteria of the genus *Bacillus,* yeasts, insect cells, insects, animal cells, and plant cells. Specific examples of bacteria of the genus *Escherichia* that may be used include *Escherichia coli* K12 DH1 (Proc. Natl. Acad. Sci. USA, 60, 160, 1996), HB101 (J. Mol. Biol., 41, 459, 1969), and C600 (Genetics, 39, 4401, 1954). Examples of bacteria of the genus *Bacillus* that may be used include *Bacillus subtilis,* MI114 (Gene, 24, 255, 1983). Examples of yeasts that may be used include *Saccharomyces cerevisiae* AH22 and AH22R-; *Schizosaccharomyces pombe* NCYC1913 and NCYC2036; and *Pichia pastoris*. Examples of insect cells that may be used when the virus used is AcNPV include a cell line derived from the larvae of *Spodoptera frugiperda* (*Spodoptera frugiperda* cells; Sf cells), MG1 cells derived from the midgut of *Trichoplusia ni,* High Five^{™} cells derived from eggs of *Trichoplusia ni, Mamestra brassicae*-derived cells, and *Estigmena acrea*-derived cells. When the virus used is BmNPV, a silkworm-derived cell line *(Bombyx mori N* cells; BmN cells) or such may be used. Examples of Sf cells that may be used include Sf9 cells (ATCC CRL 1711) and Sf21 cells (both disclosed in Vaughn J. L. et al., In Vivo, 13, 213-217 (1977)). Examples of animals cells that may be used include simian cell COS-7, Vero, Chinese hamster cell CHO (J. Exp. Med. 108, 945, (1995); hereinafter, referred to as CHO cells), a dhfr gene-deficient Chinese hamster cell CHO (Proc. Natl. Acad. Sci. USA, 77, 4216-4220, (1980); hereinafter, referred to as "CHO(dhfr-) cells"), mouse L cells, mouse AtT-20 cells, mouse myeloma cells, rat GH3, human FL cells, and amphibian cells such as *Xenopus* oocytes (Valle, et al., Nature 291, 338-340, (1981)). For plant cells, for example, cells derived from *Nicotiana tabacum* may be grown as callus culture.

Introduction of a plasmid vector can be accomplished by infection with a virus vector. In addition, introduction into prokaryotic cells, for example, bacteria of the genus *Escherichia* can be accomplished by the calcium chloride method or electroporation method, and introduction into bacteria of the genus *Bacillus* can be accomplished by a contact method using competent cells, or electroporation; introduction into eukaryotic cells, for example, *Saccharomyces cerevisiae* or such can be accomplished by the PEG method or electroporation method, and introduction into animal cells can be accomplished by the calcium phosphate method, DEAE dextran method which is a method that uses a cationic ribosome DOTAP (manufactured by Boehringer Mannheim), electroporation method, lipofection, or such.

### 4. Ligands for neuromedin U receptor 2 (FM4)

The present invention also relates to ligands for neuromedin U receptor 2 (FM4). In the present invention, a ligand refers to a substance that binds specifically to a receptor and has the ability to transmit information to cells that express the receptor. In a narrow definition, it refers to an endogenous ligand which is a substance intrinsic to the body and binds to a receptor expressed in cells and transmits information to cells through the receptor. However, a ligand of the present invention is not limited to the aforementioned naturally-occurring endogenous ligand present in hosts of the receptor-expressing cells. Ligands of the present invention include ligands, naturally-occurring compounds, and artificial compounds that have agonistic effects on the receptor of the present invention or salts thereof. Salts of the present invention are, for example, salts formed with inorganic acids (such as hydrochloric acid, phosphoric acid, hydrobromic acid, or sulfuric acid), or salts formed with organic acids (such as acetic acid, formic acid, propionic acid, fumaric acid, maleic acid, succinic acid, tartaric acid, citric acid, malic acid, oxalic acid, benzoic acid, methanesulfonic acid, or benzene sulfonic acid) but are not limited thereto. A naturally-occurring substance produced by a host other than the host itself can be used as a ligand in the present invention as long as it is a substance that has the ability to transmit information to cells expressing the receptor when it binds to or contacts the receptor. Furthermore, an artificial compound may be used as a ligand in the present invention as long as it is a substance that has the ability to transmit information to cells expressing the receptor when it binds to and contacts the receptor.

A ligand for neuromedin U receptor 2 (FM4) is, for example, a peptide or a low-molecular-weight compound having the ability to bind to a protein identical to a protein comprising the amino acid sequence of SEQ ID NO: 12, or to a protein substantially identical to a protein comprising the amino acid sequence of SEQ ID NO: 12, or salts thereof. Specific examples of the peptide include neuromedin U and peptides having agonistic activity substantially identical to that of neuromedin U. More specifically, examples of neuromedin U include a polypeptide having the amino acid sequence of Swiss Prot No. P48645 (SEQ ID NO: 14) and polypeptides substantially identical to the polypeptide having the amino acid sequence of Swiss Prot No. P48645 (SEQ ID NO: 14).

A peptide comprising an amino acid sequence with one or two or more (preferably one to ten or so, or more preferably one to five or so) amino acid deletions in an amino acid sequence comprising the amino acid sequence of SEQ ID NO: 14 is used as a peptide comprising an amino acid sequence identical to or substantially identical to the amino acid sequence of SEQ ID NO: 14. As described above, proteins comprising a modified amino acid sequence, in which one or more amino acid residues in a certain amino acid sequence is deleted, added, and/or substituted with other amino acids, are known to retain their original biological activity (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; and Dalbadie-McFarland, G. et al., Proc. Natl. Acad. Sci. USA (1982) 79, 6409-6413). A ligand peptide for neuromedin U receptor 2 (FM4) is preferably, for example, a peptide derived from a human or a non-human mammal, and is more preferably a human-derived peptide.

Examples of polypeptides substantially identical to a polypeptide comprising the amino acid sequence of SEQ ID NO: 14 include amino acid sequences having sequence identity of at least approximately 70% or more, at least approximately 75% or more, preferably approximately 80% or more, preferably approximately 85% or more, more preferably approximately 90% or more, and even more preferably approximately 95% or more with the amino acid sequence of SEQ ID NO: 14. Examples of polypeptides comprising an amino acid sequence substantially identical to the amino acid sequence of SEQ ID NO: 14 include polypeptides which comprise an amino acid sequence substantially identical to the amino acid sequence of SEQ ID NO: 14 and have properties substantially identical to a polypeptide comprising the amino acid sequence of SEQ ID NO: 14.

When comparing two sequences, amino acid sequence identity is determined by dividing the number of identical residues by the total number of residues and multiplying this by 100. Several computer programs for determining sequence identity using standard parameters, for example, Gapped BLAST or PSI-BLAST (Altschul, et. al. Nucleic Acids Res. 25, 3389-3402, 1997), BLAST (Altschul et. al., J. Mol. Biol. 215,403-410, 1990), and Smith-Waterman (Smith, et.al., J. Mol. Biol., 147, 195-197, 1981) can be used.

Examples of a polypeptide having an amino acid sequence identical or substantially identical to the amino acid sequence of SEQ ID NO: 14 of the present invention include a polypeptide which comprises an amino acid sequence identical or substantially identical to the amino acid sequence of SEQ ID NO: 14 and also has activities substantially identical to that of a polypeptide comprising the amino acid sequence of SEQ ID NO: 14. Examples of substantially identical activities include ligand binding activity and signal transduction effect, but are not limited thereto. "Substantially identical" indicates that these activities are of identical character. Therefore, activities such as ligand binding activity and signal transduction effect are preferably equivalent (for example, approximately 0.5 to 2 times), but the activity level of other activities and quantitative elements such as protein molecular weight may be different. Activities such as ligand binding activity and signal transduction effect can be measured according to known methods, for example, according to the determination methods and screening methods described later.

Examples of a polypeptide comprising an amino acid sequence identical or substantially identical to the amino acid sequence of SEQ ID NO: 14 of the present invention include a polypeptide produced as a result of modifications such as cleavage, after *in vivo* expression of a polypeptide that comprises an amino acid sequence identical or substantially identical to the amino acid sequence of SEQ ID NO: 16 and has a substantially identical activity as a polypeptide comprising the amino acid sequence of SEQ ID NO: 16.

For the polypeptides and proteins in this description, the left end is the N terminus (amino terminus) and the right end is the C terminus (carboxyl terminus) according to conventional peptide notation. The C terminus of ligand peptides for neuromedin U receptor 2 (FM4), including polypeptides comprising the amino acid sequence of SEQ ID NO: 14, may be any one of carboxyl group (-COOH), carboxylate (-COO-), amide (-CONH2), and ester (-COOR). Examples of R in the ester group that may be used include C1-6 alkyl groups such as methyl, ethyl, n-propyl, isopropyl, and *n*-butyl; C3-8 cycloalkyl groups such as cyclopentyl and cyclohexyl; C6-12 aryl groups such as phenyl and α-naphthyl; C7-14 aralkyl groups such as phenyl-C1-2 alkyl groups including benzyl and phenethyl, and α-naphthyl-C1-2 alkyl groups including α-naphthylmethyl; and pivaloyloxymethyl groups which are commonly used as oral esters.

When a ligand peptide of neuromedin U receptor 2 (FM4) of the present invention has a carboxyl group (or carboxylate) at a position other than its C terminus, those that have an amidated or esterified carboxyl group are also included in the ligand peptides of the present invention. The ester that may be used in this case is, for example, a C-terminal ester mentioned above. Furthermore, a ligand peptide of neuromedin U receptor 2 (FM4) of the present invention also includes the aforementioned peptides in which the amino group of the N-terminal methionine residue is protected by a protective group (for example, C1-6 acyl group such as C2-6 alkanoyl group, for instance, formyl group or acetyl group); the aforementioned peptides in which the glutamyl group generated through *in vivo* cleavage of the N terminus is pyroglutaminated; the aforementioned peptides in which a substituent (for example, -OH, -SH, amino group, imidazole group, indole group, or guanidino group) on a side chain of an amino acid in the molecule is protected by an appropriate protective group (for example, C1-6 acyl group such as C2-6 alkanoyl group, for instance, formyl group or acetyl group); and conjugated peptides such as the so-called glycopeptides/glycoproteins in which sugar chains are linked.

Specific examples of a ligand peptide for neuromedin U receptor 2 (FM4) include a human-derived polypeptide comprising the amino acid sequence of SEQ ID NO: 14; however, even if a polypeptide is not identical to this amino acid sequence in terms of substance, as long as it has functions or activities as a ligand of neuromedin U receptor 2 (FM4) (for example, ligand binding activity or signal transduction effect), it may be suitably used in the present invention. The ligand may be prepared by a chemical synthesis method, extracted from a naturally-occurring substance, or prepared as a recombinant protein.

When preparing the ligand as a recombinant protein, it can be prepared using the methods described in 1. to 3. above. Specific examples of a polynucleotide used in this occasion include a polynucleotide encoding the polypeptide of SEQ ID NO: 14, for example, a polynucleotide comprising the nucleotide sequence of SEQ ID NO: 13. An example of such a polynucleotide is a polynucleotide comprising the nucleotide sequence of SEQ ID NO: 15. The polynucleotide comprising the nucleotide sequence of SEQ ID NO: 15 encodes a polypeptide comprising the amino acid sequence of SEQ ID NO: 16 as an immature polypeptide. A polypeptide comprising the amino acid sequence of SEQ ID NO: 16 is produced in a recombinant cell, secreted, and then processed. As a result, a polypeptide comprising the amino acid sequence of SEQ ID NO: 14 is produced.

The polynucleotide of SEQ ID NO: 15 can be prepared by the method described in 1. above. When it is prepared by the RT-PCR method or PCR method, the synthetic DNA primers that may be suitably used are polynucleotides comprising the nucleotide sequence of any one of SEQ ID NOs: 17, 18, 19, and 20, but are not limited thereto. That is, a polynucleotide having any sequence can be suitably used, as long as it is a neuromedin U receptor 2 (FM4)-encoding DNA that has functions or activities (for example, ligand-binding activity or signal transduction effect) substantially identical to that of the sequence of SEQ ID NO: 14 or 16, and can be amplified when expressed in suitable cells. Synthetic DNA primers having specific sequences can be prepared by a solid phase synthesis method using a semi-automated synthesizer (for example, Models 392/394 or such manufactured by PE Applied Biosystems).

When amplifying a DNA that fully encodes neuromedin U receptor 2 (FM4) by the PCR method, an RNA library containing various types of mRNAs obtained from cells expressing a polypeptide comprising the amino acid sequence of SEQ ID NO: 12 can be suitably used as template. The neuromedin U receptor 2 (FM4)-expressing cells are not particularly limited, and one may suitably use cancer cell lines of the present invention, preferably Capan-1, Capan-2, CFPAC1, HPAF II, AsPC, MPANC96, su.86.86, or such which are pancreatic cancer cell lines, in addition to cells derived from the tissues of substantia nigra, medulla oblongata, pontine reticular formation, spinal cord, thalamus, hippocampus, hypothalamus, cerebral cortex, and testis.

### 5. Screening for ligands of neuromedin U receptor 2 (FM4)

The present invention provides methods of screening for ligands for neuromedin U receptor 2 (FM4), which comprise contacting a test substance with neuromedin U receptor 2 (FM4), and measuring the effect of intracellular signal transduction mediated by neuromedin U receptor 2 (FM4). Furthermore, the present invention provides ligands obtained by the methods of screening for ligands of the present invention. The screening methods of the present invention are useful in screening for cell proliferation-suppressing agents, agents for suppressing colony formation, agents for suppressing cell movement, cancer therapeutic agents, agents for suppressing cancer metastasis, and such.

A first embodiment of the screening methods of the present invention is, for example, a method of screening for a ligand of neuromedin U receptor 2 (FM4), comprising the following steps of:
(a) contacting a test substance with a polypeptide comprising the amino acid sequence of SEQ ID NO: 12 or a polypeptide functionally equivalent thereto;
(b) detecting binding between the test substance and the polypeptide comprising the amino acid sequence of SEQ ID NO: 12 or the functionally equivalent polypeptide; and
(c) selecting a test substance that binds to the polypeptide comprising the amino acid sequence of SEQ ID NO: 12 or the functionally equivalent polypeptide.

In the first embodiment, a test substance is initially contacted with a polypeptide comprising the amino acid sequence of SEQ ID NO: 12 or to a polypeptide functionally equivalent thereto. The "test substance" in the method of the present invention is not particularly limited and may be, for example, a single compound such as a naturally-occurring compound, organic compound, inorganic compound, protein, or peptide, as well as a compound library, expression products of a gene library, cell extract, cell culture supernatant, fermentative microorganism product, marine organism extract, plant extract, prokaryotic cell extract, extract of a single-cell eukaryote, or animal cell extract, but is not limited thereto. Furthermore, when necessary, the above-mentioned test substances can be used after suitable labeling. Examples of labels include radiolabels and fluorescent labels, but are not limited thereto.

"Contact" in the present invention is carried out as follows. For example, if a polypeptide comprising the amino acid sequence of SEQ ID NO: 12 or a polypeptide functionally equivalent thereto is present in a purified state, contact can be accomplished by adding a test substance to the purified sample. If the polypeptide is present in a form expressed on cell membrane or in a cell extract solution, contact can be accomplished by adding a test substance to the cell culture solution or cell extract solution, respectively. When the test substance is a protein, contact can be accomplished, for example, by introducing a vector comprising a DNA encoding the protein into a cell expressing a polypeptide comprising the amino acid sequence of SEQ ID NO: 12 or a polypeptide functionally equivalent thereto, or by adding the vector to the culture solution of cells expressing a polypeptide comprising the amino acid sequence of SEQ ID NO: 12 or a polypeptide functionally equivalent thereto. Alternatively, for example, two hybrid methods using yeast cells, animal cells, or the like can be used.

In the first embodiment, the above-mentioned binding between a test substance and a polypeptide comprising the amino acid sequence of SEQ ID NO: 12 or a functionally equivalent polypeptide is then detected. Means for detecting or measuring binding between proteins can be accomplished, for example, by using labels attached to the proteins. Examples of the type of labels include fluorescent labels and radiolabels. Furthermore, measurements can be carried out using known methods such as the enzyme two-hybrid method or measurement methods that use BIACORE. In the present method, test substances that bound to the above-mentioned biosynthetic enzyme are then selected. The selected test substances include candidate substances for pharmaceutical agents for treating cancer, and the like. The selected test substances may also be used as test substances for the screening described below.

The present invention also provides methods of screening for ligands for a polypeptide comprising the amino acid sequence of SEQ ID NO: 12 or a polypeptide functionally equivalent thereto, which comprise the following steps of:
(a) contacting a test substance with a cell expressing a polypeptide comprising the amino acid sequence of SEQ ID NO: 12 or a polypeptide functionally equivalent thereto, or with an extract of said cell;
(b) measuring cell stimulating activity of the test substance in the cell of step (a) or an extract of the cell; and
(c) selecting a test substance that alters the above-mentioned cell stimulating activity compared to when the test substance is not contacted.

The screening methods of the present invention can be suitably accomplished. In the case when the test substance is contacted with neuromedin U receptor 2 (FM4), the screening methods can be carried out by detecting the responsive reaction of neuromedin U receptor 2 (FM4) to the test substance, for example, by detecting a physiological or biochemical change of the neuromedin U receptor 2 (FM4) protein. Alternatively, in the case when the test substance is contacted with cells expressing neuromedin U receptor 2 (FM4), the screening methods can be carried out by measuring the stimulation activity or the like in the cells.

Specifically, the screening methods of the present invention are methods of screening for compounds, or salts thereof, having neuromedin U receptor 2 (FM4) protein-mediated cell-stimulating activity, which comprise the steps of contacting a test substance with a neuromedin U receptor 2 (FM4) protein expressed on cell membrane, and then measuring the neuromedin U receptor 2 (FM4) protein-mediated cell-stimulating activity. The neuromedin U receptor 2 (FM4) protein expressed on cell membrane can be obtained, for example, by culturing a transformant comprising a neuromedin U receptor 2 (FM4) protein-encoding DNA. Examples of the cell stimulating activity include inhibition of cell proliferation, colony formation assay, inhibition of cell movement, and promotion or inhibition of arachidonic acid release, acetylchloline release, intracellular Ca²⁺ release, intracellular cAMP production, intracellular cGMP production, inositol phosphate production, changes in cell membrane potential, phosphorylation of intracellular proteins, activation of c-fos, pH reduction, or such, but are not limited thereto.

Specifically, the screening methods of the present invention are methods of screening for compounds, or salts thereof, having neuromedin U receptor 2 (FM4) protein-mediated cell-stimulating activity, which comprise the steps of contacting a test substance with an extract of transformed cells comprising a DNA encoding the neuromedin U receptor 2 (FM4) protein, and then measuring the neuromedin U receptor 2 (FM4) protein-mediated cell-stimulating activity. Examples of the cell-stimulating activity include the above-mentioned activities.

Measurement of the neuromedin U receptor 2 (FM4) protein-mediated cell-stimulating activity can be carried out using, for example, cells expressing the neuromedin U receptor 2 (FM4) protein or an extract of these cells. Cell lines expressing the aforementioned recombinant neuromedin U receptor 2 (FM4) protein, or the like, are desirable as cells expressing the neuromedin U receptor 2 (FM4) protein. Neuromedin U receptor 2 (FM4) protein-expressing cells which are transformants may be stably-expressing cell lines or transiently expressing cell lines. Furthermore, the same types of cells as those described above may be used for the animal cells. Extracts of neuromedin U receptor 2 (FM4) protein-expressing cells include membrane fractions containing the protein. Examples of test substances include peptides, proteins, nonpeptidic compounds, synthetic compounds, salts of these compounds, fermentation products, cell extract, plant extract, and animal tissue extract, but are not limited thereto.

Examples of assay systems that may be used as a method for measuring cell stimulating activity mediated by the neuromedin U receptor 2 (FM4) protein include the following assay systems (1) to (9).
(1) When a receptor-expressing cell is stimulated with a receptor agonist, an intracellular G protein is activated and GTP binds to it. This phenomenon is also observed in the cell membrane fraction of receptor-expressing cells. Generally, GTP is converted to GDP by hydrolysis. If GTPγS is added to the reaction solution during this process, like GTP, GTPγS binds to the G protein, but it is not hydrolyzed and maintains the state of being bound to the cell membrane containing the G-protein. Using labeled GTPγS, it is possible to measure the activity of the receptor agonist to stimulate receptor-expressing cells by measuring the radioactivity remaining in the cell membrane. Using this reaction, the stimulating activity of the test substances and ligands of the present invention on neuromedin U receptor 2 (FM4) protein-expressing cells can be measured. This method does not use cells that contain the neuromedin U receptor 2 (FM4) protein. This method is an assay that uses a membrane fraction containing the neuromedin U receptor 2 (FM4) protein, and measures cell-stimulating activity by using the activity of promoting GTPγS binding to the neuromedin U receptor 2 (FM4) protein-containing membrane fraction as an index. In this assay, a substance that shows activity to promote GTPγS binding to the neuromedin U receptor 2 (FM4) protein-containing membrane fraction is an agonist. In the assay system of (1), the neuromedin U receptor 2 (FM4) protein-mediated cell-stimulating activity can be measured by adding a test substance and observing changes in the activity to promote GTPγS binding to the neuromedin U receptor 2 (FM4) protein-containing cell membrane fraction.
   An example of the activity assay will be specifically described below. The neuromedin U receptor 2 (FM4) protein-containing cell membrane fraction is diluted with a membrane dilution buffer solution (50 mM Tris, 5 mM MgCl₂, 150 mM NaCl, 1 µM GDP, 0.1% BSA, pH 7.4). The dilution ratio may vary depending on the expression level of the neuromedin U receptor 2 (FM4) protein. The diluted cell membrane fraction is dispensed into Falcon 2053 in aliquots of 0.2 mL, a ligand of the present invention or a test substance is added thereto, and [³⁵S]GTPγS is added to give a final concentration of 200 pM. This is kept at 25°C for an hour, then an ice-cooled buffer solution (50 mM Tris, 5 mM MgCl₂, 150 mM NaCl, 0.1 % BSA, 0.05% CHAPS, pH7.41, 5 mL) is added for washing, and this is filtered through a glass fiber filter paper GF/F. After keeping the temperature at 65°C for 30 minutes for drying, the radioactivity of [³⁵S]GTPγS bound to the membrane fraction remaining on the filtering paper is measured on a liquid scintillation counter. The radioactivity of the control when the ligand of the present invention is added is set as 100%, the radioactivity of the control without addition of the ligand of the present invention is set as 0%, and effect of the test substance on the activity to promote GTPγS binding caused by the test substance is calculated based on the value of radioactivity measured when the test substance is added.
(2) Neuromedin U receptor 2 (FM4) protein-expressing cells show decrease in the amount of intracellular cAMP when they are stimulated by a ligand of the present invention. This reaction can be utilized to measure the neuromedin U receptor 2 (FM4) protein-mediated cell-stimulating activity by a ligand of the present invention in the neuromedin U receptor 2 (FM4) protein-expressing cells. The amount of cAMP production in various animal cells expressing the neuromedin U receptor 2 (FM4) protein can be measured by RIA using anti-cAMP antibodies obtained by immunizing mice, rats, rabbits, goats, cattle, or such and ¹²⁵I-Iabeled cAMP (both are commercially available). Alternatively, other EIA systems that combine anti-cAMP antibody and labeled cAMP can also be used for the measurement. It is also possible to quantify by the SPA method using ¹²⁵I-labeled cAMP and beads containing scintillant to which an anti-cAMP antibody is fixed using Protein A, or an antibody against the IgG of the animal used to produce the anti-cAMP antibody (using the kit manufactured by Amersham Pharmacia Biotech). In an assay on inhibition of cAMP production, the change in suppression of the quantity of intracellular cAMP produced as a result of the sole administration of a ligand of the present invention or of a test substance can be measured, by increasing the quantity of intracellular cAMP using a ligand such as Calcitonin or Forskolin which increases the quantity of intracellular cAMP, and then adding the ligand of the present invention or the test substance. Through this measurement, the neuromedin U receptor 2 (FM4) protein-mediated cell-stimulating activity by the ligand of the present invention or a test substance can be calculated.
   The assay is described in more detail below. Neuromedin U receptor 2 (FM4) protein-expressing CHO cells (DG44 cells; Example 2-2 which is described later) are seeded at 5 x 10⁴ cells/well into a 24-well plate, and cultured for 48 hours. The cells are washed with Hanks' buffer (pH 7.4) containing 0.2 mM 3-isobutyl-methylxanthine, 0.05% BSA, and 20 mM HEPES (hereinafter, Hanks' (pH 7.4) buffer containing 0.2 mM 3-isobutyl-methylxanthine, 0.05% BSA, and 20 mM HEPES will be referred to as the reaction buffer). Then, 0.5 mL of the reaction buffer is added, and the cells are kept warm in an incubator for 30 minutes. The reaction buffer is then removed and 0.25 mL of fresh reaction buffer is added to the cells. Then, 0.25 mL of the reaction buffer containing 2 µM Forskolin in addition to 1 nM of a ligand of the present invention or a test substance is added to the cells, and reacted at 37°C for 24 minutes. 100 µL of 20% perchloric acid is added to stop the reaction. Then, intracellular cAMP is extracted through one hour of incubation on ice. The quantity of cAMP in the extract solution is measured using a cAMP EIA kit (Amersham Pharmacia Biotech). The amount of cAMP produced by the Forskolin stimulation is set as 100%, the amount of cAMP suppressed by the addition of 1 nM of the ligand of the present invention is set as 0%, and the activity of the test substance S to suppress cAMP production is calculated. To measure the cAMP production-promoting activity, a test substance is added to CHO cells expressing the protein of the present invention without Forskolin, and cAMP production is quantified according to the above-mentioned method.
(3) A DNA containing CRE (cAMP response element) is inserted into the multi-cloning site upstream of the luciferase gene of the PicaGene basic vector or PicaGene enhancer vector (Toyo Ink). This is referred to as CRE-reporter gene vector. In cells transfected with the CRE-reporter gene vector, a stimulation accompanied by cAMP increase induces expression of the luciferase gene through CRE and subsequent production of the luciferase protein. That is, by measuring the luciferase activity, it is possible to detect the change in the quantity of cAMP in the cells into which the CRE-reporter gene vector has been introduced. The neuromedin U receptor 2 (FM4) protein-mediated cell-stimulating activity by a ligand of the present invention or a test substance can be measured using cells produced by transfecting neuromedin U receptor 2 (FM4) protein-expressing cells with the CRE-reporter gene vector.
   The assay is described in detail below. Neuromedin U receptor 2 (FM4) protein-expressing cells into which the CRE-reporter gene has been introduced are seeded into a 24-well plate at a concentration of 5 x 10³ cells/well, and cultured for 48 hours. The cells are washed with Hanks' buffer (pH 7.4) containing 0.2 mM 3-isobutyl-methylxanthine, 0.05% BSA, and 20 mM HEPES, which is hereinafter referred to as the reaction buffer. Then, 0.5 mL of the reaction buffer is added, and the cells are kept warm in an incubator for 30 minutes. The reaction buffer is then removed and 0.25 mL of fresh reaction buffer is added to the cells. Then, 0.25 mL of the reaction buffer that contains 2 µM Forskolin in addition to 1 nM of a peptide of the present invention, or 1 nM of a peptide of the present invention and a test substance is added to the cells, and reacted at 37°C for 24 minutes. The cells are dissolved in a cell lysis agent for PicaGene (Toyo Ink Mfg. Co., Ltd.), and a luminescence substrate (Toyo Ink Mfg. Co., Ltd.) is added to the lysate. Luminescence by luciferase can be measured by, without limitation, a luminometer, a liquid scintillation counter, or a top counter. The cell-stimulating activity mediated by neuromedin U receptor 2 (FM4) protein and the ligand of the present invention or the test substance can be measured using suppression of the level of luciferase luminescence as an index. More specifically, the suppressive effect of the ligand of the present invention or the test substance on the luminescence level which increased due to Forskolin stimulation can be used as an index to measure the neuromedin U receptor 2 (FM4) protein-mediated cell-stimulating activity of the ligand of the present invention or the test substance. In addition to luciferase, alkaline phosphatase, chloramphenicol acetyltransferase, β-galactosidase, or such may be employed as the reporter gene, but it is not limited thereto. The enzymatic activity of gene products from these reporter genes can be readily measured using the commercially available assay kits described below. Alkaline phosphatase activity can be measured using, without limitation, for example, Lumi-Phos530 manufactured by Wako Pure Chemical Industries. Chloramphenicol acetyltransferase activity can be measured using, without limitation, for example, FAST CAT chloramphenicol acetyltransferase Assay Kit manufactured by Wako Pure Chemical Industries. β-Galactosidase activity can be measured using, without limitation, for example, Aurora Gal-XE manufactured by Wako Pure Chemical Industries.
(4) As a result of stimulation by a ligand of the present invention, Neuromedin U receptor 2 (FM4) protein-expressing cells release arachidonic acid metabolites to the outside of the cells. By incorporating radioactive arachidonic acid into the cells in advance, the neuromedin U receptor 2 (FM4) protein-mediated cell-stimulating activity can be measured using the radioactivity released to the outside of the cells as an index. That is, the neuromedin U receptor 2 (FM4) protein-mediated cell-stimulating activity by a ligand of the present invention or a test substance can be measured by examining the effects of addition of the ligand of the present invention or the test substance on arachidonic acid metabolite-releasing activity.
   The assay for the neuromedin U receptor 2 (FM4) protein-mediated cell-stimulating activity is described in detail below. CHO cells expressing the protein of the present invention are seeded into a 24-well plate at 5 x 10⁴ cells/well. After 24 hour cultivation, [³H] arachidonic acid is added at 0.25 µCi/well. Sixteen hours after the addition of [³H] arachidonic acid, the cells are washed with Hanks' buffer (pH 7.4) containing 0.05% BSA, and 20 mM HEPES. To each well is added 500 µL of a buffer containing 10 nM (final concentration) of a peptide of the present invention, or 10 nM (final concentration) of a ligand of the present invention or a test substance dissolved in Hanks' buffer (pH 7.4) containing 0.05% BSA and 20 mM HEPES. Hereinafter the reaction buffer refers to Hanks' buffer (pH 7.4) containing 0.05% BSA and 20 mM HEPES. After incubating at 37°C for 60 minutes, 400 µL of the reaction solution is added to a scintillator and the amount of [³H] arachidonic acid metabolites released in the reaction solution is measured using a scintillation counter. By setting the amount of [³H] arachidonic acid metabolites in the medium obtained with a reaction buffer that does not contain the ligand of the present invention as 0%, and the amount of [³H] arachidonic acid metabolites in the medium obtained when 10 nM of the ligand of the present invention was added as 100%, the neuromedin U receptor 2 (FM4) protein-mediated cell-stimulating activity by the test substance can be calculated by measuring the amount of [³H] arachidonic acid metabolites in the medium obtained when the test substance was added.
(5) When neuromedin U receptor 2 (FM4) protein-expressing cells are stimulated by a ligand of the present invention, their intracellular Ca²⁺ ion concentration increases. Using this phenomenon, effects of a ligand of the present invention or a test substance on the neuromedin U receptor 2 (FM4) protein-mediated cell-stimulating activity can be investigated.
   Specifically, the investigation can be carried out by a method that follows Example 2-3 described later. Neuromedin U receptor 2 (FM4) protein-expressing cells are seeded into a 96-well plate (black plate for fluorescence measurements) at 2 x 10⁴ cells/well, and then cultured overnight. After removing the medium, a Fluo 4 AM solution [assay buffer (2 mM HEPES, and 1.5 mM probenecid in HBSS) containing 2% FCS, 3 µM Fluo 4 AM (Molecular PROBES)] is added at 50 µL/well. After incubating the cells for 30 minutes at dark at 37°C, the cells are washed three times in the assay buffer. Then, an assay buffer containing 2.5 µM of NmU (Funakoshi) is added, and intracellular Ca²⁺-dependent fluorescence trace at 490-nm excitation is monitored on a fluorescence analysis plate reader (Fusion; Perkin Elmer). The neuromedin U receptor 2 (FM4) protein-mediated cell-stimulating activity can be measured by observing the increase in fluorescence intensity as a result of addition of the ligand of the present invention or the test substance. In another embodiment, the gene of a protein that luminesces in association with increase in intracellular Ca²⁺ ion (for example, aequorin) is coexpressed in the neuromedin U receptor 2 (FM4) protein-expressing cells, and the luminescence following conversion of the protein gene (for example, aequorin) to a Ca²⁺-bound form as a result of the increase in intracellular Ca²⁺ ion concentration is used. The neuromedin U receptor 2 (FM4) protein-mediated cell-stimulating activity can be determined by measuring the difference in luminescence intensity which changes depending on whether the ligand of the present invention or the test substance is added.
(6) Besides the method of coexpressing the gene of a protein that luminesces due to increase in intracellular Ca²⁺ ions (for example, aequorin), a method that coexpresses a DNA in which a reporter gene is inserted downstream of a transcription element (for example, TRE (TPA response element)) that responds to increase in intracellular Ca²⁺ ions can also be suitably used. That is, the TRE (TPA response element)-containing DNA is inserted into the multi-cloning site upstream of the luciferase gene of the PicaGene basic vector or PicaGene enhancer vector (Toyo Ink Mfg. Co., Ltd.). This will be referred to as TRE-reporter gene vector. In cells transfected with the TRE-reporter gene vector, the resultant stimulation of the increase in the intracellular Ca²⁺ ion concentration induces expression of the luciferase gene through TRE and subsequent production of the luciferase protein. That is, by measuring the luciferase activity, changes in the amount of intracellular calcium ion in the cells into which the TRE-reporter gene vector has been introduced can be detected. Accordingly, neuromedin U receptor 2 (FM4) protein-mediated cell-stimulating activity can be measured by administering a ligand of the present invention or a test substance to cells prepared by transfecting the TRE-reporter gene vector into the neuromedin U receptor 2 (FM4) protein-expressing cells and observing the increase in luminescence.
   The assay is specifically described below. Neuromedin U receptor 2 (FM4) protein-expressing cells into which the TRE-reporter gene has been introduced are seeded into a 24-well plate at 5 x 10³ cells/well and cultured for 48 hours. The cells are washed with Hanks' buffer (pH 7.4) containing 0.05% BSA and 20 mM HEPES. Then, 10 nM of a ligand of the present invention or a test substance is added, and then this is allowed to react at 37°C for 60 minutes. The cells are dissolved with a cell lysis agent for PicaGene (Toyo Ink Mfg. Co., Ltd.), and a luminescence substrate (Toyo Ink Mfg. Co., Ltd.) is added to the lysate. Luciferase luminescence can be measured by, without limitation, a luminometer, a liquid scintillation counter, or a top counter. The method utilizes the phenomena in which administration of the ligand of the present invention increases the intracellular Ca²⁺ ion concentration, and increases the level of luminescence. By setting the level of luminescence observed when using a reaction buffer not containing the ligand of the present invention as 0% and the level of luminescence observed when the ligand of the present invention was added at 10 nM as 100%, the neuromedin U receptor 2 (FM4) protein-mediated cell-stimulating activity by the ligand of the present invention or the test substance can be calculated by measuring the luminescence level observed when the test substance was added. In addition to luciferase, alkaline phosphatase, chloramphenicol acetyltransferase, β-galactosidase, or such may be employed as the reporter gene. The enzymatic activity of gene products from these reporter genes can be readily measured using the commercially available assay kits described below. Alkaline phosphatase activity can be measured using, for example, Lumi-Phos530 manufactured by Wako Pure Chemical Industries, without limitation thereto. Chloramphenicol acetyltransferase activity can be measured, for example, using the FAST CAT chloramphenicol acetyltransferase Assay Kit manufactured by Wako Pure Chemical Industries, but without limitation thereto. β-Galactosidase activity can be measured, for example, using Aurora Gal-XE manufactured by Wako Pure Chemical Industries, but without limitation thereto.
(7) According to the present invention, the neuromedin U receptor 2 (FM4) protein-mediated cell-stimulating activity can be measured using the proliferation-suppressing effect on neuromedin U receptor 2 (FM4) protein-expressing cells as an index. A general cell proliferation activity assay can be suitably used for evaluating the suppression of proliferation of neuromedin U receptor 2 (FM4) protein-expressing cells. That is, the following can be suitably used: (a) a method of measuring the number of cells on a hemocytometer; (b) a method which involves adding to cultured cells [³H]-thymidine, which is a precursor for DNA replication, produced by radiolabeling thymidine; washing and removing the free [³H]-thymidine in the medium after a certain time has lapsed, and then measuring the amount of radioactivity incorporated into the cells using a liquid scintillation counter; (c) a method that uses the phenomena in which the tetrazolium salt compound is converted to a compound displaying color of a particular wavelength by succinate dehydrogenase found in cellular mitochondria (method of measuring the intensity of this coloration using a spectrophotometer); and such. The dye exclusion method is used as a modified method of (a), and it allows live and dead cells to be differentially detected rather than simply measuring the number of cells, wherein a compound such as Trypan blue, which has a property of being excluded to the outside of the cell by live cells, is added when measuring the number of cells. In addition to WST-8 (Japanese Patent No. 2757348) used in Example 2-4 of this application, compounds that may be suitably used as the tetrazolium salt of (c) are specifically, for example, WST-1 (Biochem., 179, 1-7 (1989)), MTT (J. Immunol. Methods 65, 55-63, (1983)), MTS (Cancer Commun. 3, 207-212, (1991)), and XTT (Cancer Res. 48, 4827-4833, (1988)).
   More specifically, the neuromedin U receptor 2 (FM4) protein-mediated cell-stimulating activity can be measured by using a proliferation-suppressing effect on neuromedin U receptor 2 (FM4) protein-expressing cells as an index, with methods such as those described below. Neuromedin U receptor 2 (FM4) protein-expressing cells of the present invention are seeded into a 96-well plate, and 48 hours later, a coloration reagent containing WST-8 as the major ingredient (Cell Count Reagent SF, Nakalai Tesque) is added to the cells, and intensity of the displayed color is measured using a plate reader. By setting the coloration value observed when a reaction buffer not containing the ligand of the present invention is added as 0%, and the coloration value observed when the ligand of the present invention is added as 100%, the neuromedin U receptor 2 (FM4) protein-mediated cell-stimulating activity by the test substance can be calculated by measuring the coloration value observed when the test substance is added.
(8) Furthermore, according to the present invention, the neuromedin U receptor 2 (FM4) protein-mediated cell-stimulating activity can be measured using the colony formation-suppressing effect on neuromedin U receptor 2 (FM4) protein-expressing cells as an index. Malignant cell transformation is induced by genetic and epigenetic changes, and produces a cell population that grows on its own regardless of signals from growth-suppressing factors and can grow with hardly any need for extracellular growth factors. Specifically, transformed cells such as cancer cells are different from normal cells in that they can proliferate without adhesion (anchorage-independent growth). That is, normal cells that proliferate by adhering to a substrate cannot proliferate in soft agar since there is no anchorage; in contrast, since transformed cells such as cancer cells can undergo anchorage-independent growth, they have the ability to form colonies in soft agar. More specifically, the activity of a test neuromedin U receptor 2 (FM4) protein-expressing cell to form colonies in soft agar can be used as an index to evaluate the degree of transformation of the cell, its metastatic potential, and such. Furthermore, the colony forming ability can be evaluated as the neuromedin U receptor 2 (FM4) protein-mediated cell-stimulating activity, based on the presence or absence of a test substance or a ligand for the neuromedin U receptor 2 (FM4) protein in the soft agar used for the measurement.
   Specifically, a method such as the following can be used to measure the neuromedin U receptor 2 (FM4) protein-mediated cell-stimulating activity by sing the colony forming ability of neuromedin U receptor 2 (FM4) protein-expressing cells as an index. More specifically, base agar (0.5% agar, 1x MEM, and 10% FCS) is added to each well of a 6-well plate at 1.5 mL/well, and then 1.5 mL/well (0.5 x 10⁴ cells/well) of PANC1 cells or FM4-PANC1 cells are added to each of the wells in the presence or absence of the ligand of the present invention or a test substance in top agar (0.35% agar, 1x MEM, and 10% FCS). After culturing for one month, the number of colony forming cells in the soft agar is counted under a microscope. By setting the number of colonies observed when a reaction buffer not containing the ligand of the present invention was added as 100%, and the number of colonies observed when the ligand of the present invention was added as 0%, the neuromedin U receptor 2 (FM4) protein-mediated cell-stimulating activity due to the test substance can be calculated by measuring the number of colonies observed when the test substance was added.
(9) Furthermore, according to the present invention, the neuromedin U receptor 2 (FM4) protein-mediated cell-stimulating activity can be measured using the cell movement-suppressing effect on neuromedin U receptor 2 (FM4) protein-expressing cells as an index. In the case of normal cells, when cells contact each other (contact inhibition), inhibition of movement takes place due to proliferation, and when cells proliferate densely, they align parallel to each other. In contrast, in cells that have been malignantly transformed by genetic or epigenetic changes, inhibition of movement due to contact between cells does not take place (loss of contact inhibition), and cells show a proliferation image indicative of irregular alignment where the cells overlap with each other. Evaluation of such contact inhibition phenomena of cell movement enables evaluation of the degree of transformation, metastatic potential, and such of a test cell. That is, by using the cell movement activity of a test neuromedin U receptor 2 (FM4) protein-expressing cell as an index, the degree of transformation, metastatic potential, and such of the cell can be evaluated. Furthermore, by detecting the cell movement activity based on the presence or absence of a ligand or a test substance, the neuromedin U receptor 2 (FM4) protein-mediated cell-stimulating activity can be evaluated.
   Specifically, methods such as the wound healing assay described below can be used to measure the neuromedin U receptor 2 (FM4) protein-mediated cell-stimulating activity by using the cell movement activity of neuromedin U receptor 2 (FM4) protein-expressing cells as an index. Specifically, neuromedin U receptor 2 (FM4) protein-expressing cells grown to confluency in a plate are wounded with a pipette tip to make a fixed clearance between the cells. After washing the cells twice with PBS, the cells are cultured in the presence or absence of a ligand of the present invention or a test substance. Similarly, cells that do not express the neuromedin U receptor 2 (FM4) protein are cultured under the same conditions as a control. Twenty-four hours later, presence of cells that moved into the clearance between the cells is observed under a microscope, and their number is counted. By setting the number of cells observed when a reaction buffer not containing the ligand of the present invention was added as 0%, and the number of cells observed when the ligand of the present invention was added as 100%, the neuromedin U receptor 2 (FM4) protein-mediated cell-stimulating activity of the test substance can be calculated by counting the number of colonies observed when the test substance was added.

### 6. Screening of ligands for neuromedin U receptor 2 (FM4) by competition assay

The present invention also provides methods of screening for a ligand for a polypeptide comprising the amino acid sequence of SEQ ID NO: 12 or a polypeptide functionally equivalent thereto, which comprise the following steps of:
(a) contacting a test substance with a cell, or an extract of the cell, expressing the polypeptide comprising the amino acid sequence of SEQ ID NO: 12 or the polypeptide functionally equivalent thereto;
(b) measuring cell-stimulating activity of the test substance for the cell of step (a) or the extract of the cell; and
(c) selecting a test substance that alters the above-mentioned cell stimulating activity as compared to when neuromedin U is contacted.

In another preferred embodiment of the screening of the present invention, screening by competition assay can be suitably carried out by measuring and comparing, for example, the amount of the ligand of the present invention bound to neuromedin U receptor 2 (FM4) when (i) the ligand of the present invention was contacted with neuromedin U receptor 2 (FM4), and when (ii) the ligand of the present invention and a test substance were contacted with the above-mentioned neuromedin U receptor 2 (FM4). The cell-stimulating activity of compounds found by this screening method can be further evaluated, based on a method similar to the above-described method of screening for compounds having a cell-stimulating activity or salts thereof.

An embodiment of the above-described competition assay screening method is, for example, a method of screening for a compound that alters the binding between a ligand of the present invention and a neuromedin U receptor 2 (FM4) protein, or a salt thereof, in which the method comprises the steps of:
(1) contacting a labeled ligand of the present invention with a neuromedin U receptor 2 (FM4) protein;
(2) contacting a labeled ligand of the present invention and a test substance with a neuromedin U receptor 2 (FM4) protein; and
(3) measuring the amount of labeled ligand of the present invention bound to the protein of the present invention in each of the above-mentioned steps (1) and (2), and then comparing the bound amount of steps (1) and (2).

An embodiment of the competition assay screening method of the present invention is, for example, a method of screening for a compound that alters the binding between a ligand of the present invention and a neuromedin U receptor 2 (FM4) protein, or a salt thereof, in which the method comprises the steps of:
(1) contacting a labeled ligand of the present invention with a cell comprising a neuromedin U receptor 2 (FM4) protein or with a membrane fraction of the cell;
(2) contacting a labeled ligand of the present invention and a test substance with a cell comprising a neuromedin U receptor 2 (FM4) protein or with a membrane fraction of the cell; and
(3) measuring the amount of labeled ligand of the present invention bound to the cell or the membrane fraction in each of the above-mentioned steps (1) and (2), and then comparing the bound amount of steps (1) and (2).

An embodiment of the competition assay screening method of the present invention is, for example, a method of screening for a compound that alters the binding between a ligand of the present invention and a neuromedin U receptor 2 (FM4) protein, or a salt thereof, in which the method comprises the steps of:
(1) contacting a labeled ligand of the present invention with a neuromedin U receptor 2 (FM4) protein expressed on cell membrane by culturing a transformant containing a DNA encoding a neuromedin U receptor 2 (FM4) protein;
(2) contacting a labeled ligand of the present invention and a test substance with a neuromedin U receptor 2 (FM4) protein expressed on cell membrane by culturing a transformant containing a DNA encoding a neuromedin U receptor 2 (FM4) protein; and
(3) measuring the amount of labeled ligand of the present invention bound to the neuromedin U receptor 2 (FM4) protein in each of the above-mentioned steps (1) and (2), and then comparing the bound amount of steps (1) and (2).

Specific description of the screening methods of the present invention is as follows. First, any substance may be used for the neuromedin U receptor 2 (FM4) protein used in the screening method of the present invention, as long as it contains the above-mentioned neuromedin U receptor 2 (FM4) protein. However, organs derived from humans in particular are very difficult to obtain, and thus expressing the neuromedin U receptor 2 (FM4) protein in a large scale using recombinants is suitable. To produce the neuromedin U receptor 2 (FM4) protein, the aforementioned methods or such are used. When cells or cell membrane fraction containing the neuromedin U receptor 2 (FM4) protein are used in the screening methods of the present invention, the preparation method described below can be used. When cells containing the neuromedin U receptor 2 (FM4) protein are used, the cells may be fixed using glutaraldehyde, formalin, or the like. The method of fixing can be performed by a known method. The cells containing the neuromedin U receptor 2 (FM4) protein include host cells that express the neuromedin U receptor 2 (FM4) protein. Such host cells include the above-described *Escherichia coli, Bacillus subtilis*, yeast, insect cells, and animal cells. The membrane fraction refers to a fraction abundant in cell membrane, obtained after cell disruption by a known method. The cell disruption methods include a method in which the cells are crushed using a Potter-Elvehjem-type homogenizer, disruption using a Waring blender or Polytron (produced by Kinematica Inc.), disruption by ultrasound, and disruption by spraying cells through narrow nozzles while applying pressure using a French press or the like. Cell membrane fractionation is carried out mainly by using fractionation that uses centrifugal force, such as centrifugal fractionation and density gradient centrifugation. For example, after the disrupted cell solution is centrifuged at a low speed (500 rpm to 3,000 rpm) for a short period of time (normally about 1 to 10 minutes), the supernatant is further centrifuged at a high speed (15,000 rpm to 30,000 rpm) normally for 30 minutes to two hours. The precipitate thus obtained is used as the membrane fraction. The membrane fraction is rich in the expressed protein of the present invention, and membrane components, such as cell-derived phospholipids and membrane proteins. The amount of the neuromedin U receptor 2 (FM4) protein in the cell and the membrane fraction containing the neuromedin U receptor 2 (FM4) protein is preferably 10³ to 10⁸ molecules per cell, and more preferably 10⁵ to 10⁷ molecules per cell. As the level of expression increases, the ligand binding activity per membrane fraction (specific activity) increases; thus, one can not only construct a highly sensitive screening system, but also assay large quantities of samples using the same lot.

To perform the aforementioned competition assay screening which screens for a compound that alters the binding between a ligand of the present invention and neuromedin U receptor 2 (FM4), an appropriate neuromedin U receptor 2 (FM4) protein fraction and a labeled ligand of the present invention are used. The fraction containing the neuromedin U receptor 2 (FM4) protein is desirably a fraction containing a naturally-occurring neuromedin U receptor 2 (FM4) protein or a fraction containing a recombinant neuromedin U receptor 2 (FM4) protein having an activity equivalent to that of the natural protein. Herein, an equivalent activity refers to an equivalent ligand binding activity or the like. For the labeled ligand of the present invention, for example, a ligand of the present invention labeled with [³H], [¹²⁵I], [¹⁴C], [³⁵S], or such may be used. Specifically, a labeled ligand of the present invention prepared by a known method using a Bolton-Hunter reagent may also be used. More specifically, to screen for a compound that alters the binding between the neuromedin U receptor 2 (FM4) protein and a ligand of the present invention, first, a receptor preparation is prepared by suspending cells or cell membrane fractions containing the neuromedin U receptor 2 (FM4) protein in a buffer appropriate for the screening. Any buffer that does not inhibit the ligand/receptor binding can be used, and such buffers include a phosphate buffer or a Tris-HCl buffer having a pH of 4 to 10 (desirably a pH of 6 to 8). For the purpose of reducing non-specific binding; a surfactant such as CHAPS, Tween-80 (Kao-Atlas Inc.), digitonin, or deoxycholate, may be added to the buffer. Furthermore, for the purpose of suppressing degradation of the protein of the present invention or the peptide of the present invention by proteases, a protease inhibitor such as PMSF, leupeptin, E-64 (Peptide Institute, Inc.), or pepstatin may also be added. A given amount (5,000 cpm to 500,000 cpm) of the labeled ligand of the present invention is added to 0.01 mL to 10 mL of the receptor solution. At the same time, 10⁻⁴ µM to 10⁻¹ µM of the test substance is made to coexist in this mixture. To determine the amount of non-specific binding (NSB), a reaction tube containing a large excess of unlabeled ligand of the present invention is also prepared. The reaction is carried out at 0°C to 50°C, or desirably at 4°C to 37°C for 20 minutes to 24 hours, or desirably 30 minutes to three hours. After completion of the reaction, the reaction mixture is filtered through a glass fiber filter paper or the like, and washed with an appropriate amount of the same buffer. Then, residual radioactivity on the glass fiber filter paper is measured with a liquid scintillation counter or a γ-counter. When the count (B0-NSB) obtained by subtracting the amount of non-specific binding (NSB) from the count obtained in the absence of any competitive substance (B0) is set as 100%, a test substance which gives a specific binding amount (B-NSB) of, for example, 50% or less can be selected as a candidate substance having competitive inhibition ability. Moreover, as a method for measuring the binding between a ligand of the present invention and a neuromedin U receptor 2 (FM4) protein, BIAcore (manufactured by Amersham Pharmacia Biotech) may also be used. In this method, a ligand of the present invention is immobilized to a sensor chip by an amino coupling method according to the protocol attached to the device. A test substance in a buffer solution such as phosphate buffer or Tris buffer, and a cell containing a neuromedin U receptor 2 (FM4) protein; a neuromedin U receptor 2 (FM4) protein or a membrane fraction containing a neuromedin U receptor 2 (FM4) protein purified from transformants with a DNA encoding a neuromedin U receptor 2 (FM4) protein; or a purified neuromedin U receptor 2 (FM4) protein or membrane fraction containing a neuromedin U receptor 2 (FM4) protein is made to flow over the top of the sensor chip at 2-20 µL/min. Screening for a compound that alters the binding between the neuromedin U receptor 2 (FM4) protein and the ligand of the present invention can be carried out by observing whether the co-existing test substance can alter the surface plasmon resonance change caused by the binding of the neuromedin U receptor 2 (FM4) protein to the ligand of the present invention on the sensor chip. This method also allows the same measurement to be made by immobilizing the neuromedin U receptor 2 (FM4) protein to the sensor chip, and flowing a buffer solution such as phosphate buffer or Tris buffer that contains the ligand of the present invention, or the ligand of the present invention and the test substance, over the top of the sensor chip. These test substances are, for example, peptides, proteins, nonpeptidic compounds, synthetic compounds, fermentation products, cell extract, plant extract, and animal tissue extract, but are not limited thereto.

To measure the neuromedin U receptor 2 (FM4) protein-mediated cell-stimulating activity for the ligands of the present invention selected by the above-mentioned competition assay screening method, cells expressing the neuromedin U receptor 2 (FM4) protein are suitably used. The cells expressing the neuromedin U receptor 2 (FM4) protein are desirably the aforementioned neuromedin U receptor 2 (FM4) protein-expressing recombinant cell line, and the like. The transformant neuromedin U receptor 2 (FM4) protein-expressing cells may be stably-expressing cell lines or transiently-expressing cell lines. Furthermore, the same types of cells as those described above are used for the animal cells. Examples of test substances include peptides, proteins, nonpeptidic compounds, synthetic compounds, fermentation products, cell extract, plant extract, and animal tissue extract, but are not limited thereto.

### 7. Therapeutic and/or preventive agents for cancer, including pancreatic cancer, in which neuromedin U receptor 2 (FM4) is involved

In another perspective, the present invention provides pharmaceutical compositions comprising a ligand of the neuromedin U receptor 2 (FM4) protein as an active ingredient. The present invention also provides pharmaceutical agents comprising a ligand of the neuromedin U receptor 2 (FM4) protein as an active ingredient. Examples of the pharmaceutical agents of the present invention include cell proliferation-suppressing agents, agents for suppressing colony formation, agents for suppressing cell movement, cancer therapeutic agents, and agents for suppressing cancer metastasis. The pharmaceutical agents of the present invention are preferably administered to a subject affected with cancer, a subject whose cancer was removed by surgery, or a subject who is likely to be affected with cancer. The subject is, for example, a mammal (such as human, rat, mouse, guinea pig, rabbit, sheep, pig, dog, and monkey), without being limited thereto. In the present invention, "suppress" means that a ligand of the neuromedin U receptor 2 (FM4) protein decreases the biological activity of the receptor. In the present invention, the degree of this decrease is not particularly limited, and even if the biological activity is partially decreased, it is included in the meaning of "suppress" of the present invention.

In the present invention, cell proliferation-suppressing agents which comprise a ligand of the neuromedin U receptor 2 (FM4) protein as an active ingredient can also be expressed as methods for suppressing cell growth which comprise the step of administering to a subject a ligand of the neuromedin U receptor 2 (FM4) protein, or as use of a ligand of the neuromedin U receptor 2 (FM4) protein in producing cell proliferation-suppressing agents. Furthermore, in the present invention, agents for suppressing colony formation which comprise a ligand of the neuromedin U receptor 2 (FM4) protein as an active ingredient can also be expressed as methods for suppressing colony formation which comprise the step of administering to a subject a ligand of the neuromedin U receptor 2 (FM4) protein, or as use of a ligand of the neuromedin U receptor 2 (FM4) protein in producing agents for suppressing colony formation. Furthermore, in the present invention, agents for suppressing cell movement which comprise a ligand of the neuromedin U receptor 2 (FM4) protein as an active ingredient can also be expressed as methods for suppressing cell movement which comprise the step of administering to a subject a ligand of the neuromedin U receptor 2 (FM4) protein, or as use of a ligand of the neuromedin U receptor 2 (FM4) protein in producing agents for suppressing cell movement.

In the present invention, cancer therapeutic agents which comprise a ligand of the neuromedin U receptor 2 (FM4) protein as an active ingredient can also be expressed as methods for preventing or treating cancer which comprise the step of administering to a subject a ligand of the neuromedin U receptor 2 (FM4) protein, or as use of a ligand of the neuromedin U receptor 2 (FM4) protein in producing cancer therapeutic agents. Furthermore, in the present invention, agents for suppressing cancer metastasis which comprise a ligand of the neuromedin U receptor 2 (FM4) protein as an active ingredient can also be expressed as methods for preventing or treating cancer metastasis comprising the step of administering to a subject a ligand of the neuromedin U receptor 2 (FM4) protein, or as use of a ligand of the neuromedin U receptor 2 (FM4) protein in producing agents for suppressing cancer metastasis. In the present invention, the term "metastasis" refers to a phenomenon observed *in vivo* and *in vitro*, in which a cell moves away from the primary site where it formed a colony to a different site to form a different colony. The cancers targeted by the cancer therapeutic agents or agents for suppressing cancer metastasis in the present invention are not particularly limited, and may be any cancer including lung cancer, colon cancer, pancreatic cancer, and stomach cancer. An example of cancer that is preferred is pancreatic cancer.

In the present invention, the phrase "containing as an active ingredient a ligand for the neuromedin U receptor 2 (FM4) protein" means containing a ligand for the neuromedin U receptor 2 (FM4) protein as the main active ingredient, and does not limit the content ratio of the ligand of the neuromedin U receptor 2 (FM4) protein.

The ligand contained in a pharmaceutical composition of the present invention (for example, cell proliferation-suppressing agent, colony formation-suppressing agent, cell movement-suppressing agent, cancer therapeutic agent, and cancer metastasis-suppressing agent. Same below.) is not particularly limited, as long as it binds to a neuromedin U receptor 2 (FM4) protein and has cell-stimulating activity, and examples include ligands described in this description.

Ligands of the present invention may be used, for example, orally, as tablets, capsules, elixirs, or microcapsules which are sugar-coated as necessary,; or parenterally, in the form of injections of sterile solutions, suspensions, or such prepared with water or other pharmaceutically acceptable solvents. For example, ligands of the present invention may be formulated by combining them with physiologically acceptable carriers, flavoring agents, excipients, vehicles, preservatives, stabilizers, binding agents, and such, and mixing them in a unit dosage form required for generally accepted pharmaceutical practice. The amount of active ingredient in these formulations is such that appropriate doses within indicated ranges are achieved. Additives that can be mixed into tablets, capsules, and such include, for example, binding agents such as gelatin, cornstarch, tragacanth, and gum arabic; excipients such as crystalline cellulose; swelling agents such as cornstarch, gelatin, and alginic acid; lubricants such as magnesium stearate; sweeteners such as sucrose, lactose, and saccharine; and flavoring agents such as peppermint, Akamono oil, and cherry.

When the unit dosage form is a capsule, liquid carriers such as oils and fats can be further included in the types of materials mentioned above. Sterile compositions for injections can be formulated according to general formulation protocols such as dissolving or suspending an active substance, naturally produced plant oils such as sesame oil, or palm oil, or such in a vehicle such as water used for injection. Aqueous solutions used for injections include, for example, physiological saline and isotonic solutions containing glucose or other auxiliary agents (for example, D-sorbitol, D-mannitol, and sodium chloride). They may also be used in combination with appropriate solubilizing agents such as alcohol (for example, ethanol), polyalcohol (for example, propylene glycol or polyethylene glycol), or non-ionic detergent (for example, polysorbate 80^{™} or HCO-50). Oil solutions include sesame oils and soybean oils, and can be used in combination with solubilizing agents such as benzyl benzoate or benzyl alcohol. They may also be combined with buffers (for example, phosphate buffer solutions or sodium acetate buffer solutions), analgesics (for example, benzalkonium chloride or procaine hydrochloride), stabilizers (for example, human serum albumin or polyethylene glycol), preservatives (for example, benzyl alcohol or phenol), anti-oxidants, or the like. The prepared injections are typically packaged into appropriate ampules.

Since formulations obtained in this manner are safe and have low toxicity, they can be administered, for example, to a mammal (such as human, rat, mouse, guinea pig, rabbit, sheep, pig, dog, or monkey). The dose of a ligand of the present invention varies depending on the targeted disease, the target of administration, the administration route, and such, but for an ordinary adult (with a body weight of 60 kg), a ligand of the present invention is administered at approximately 1 mg to 1000 mg, or preferably approximately 5 mg to 500 mg, or more preferably approximately 10 mg to 200 mg per day. When administered parenterally, a single dose of a ligand of the present invention varies depending on the age, difference in the symptoms, target of administration, targeted disease, and such; however, when administering a ligand of the present invention to an adult (with a body weight of 60 kg) in the form of an injection, it is convenient to administer the peptide at a dose of approximately 1 mg to 1000 mg or so, preferably approximately 5 mg to 200 mg or so, or more preferably approximately 10 mg to 100 mg or so per day by injection to the affected site. For other animals, an amount converted from the amount for 60 kg can be administered.

As described above, methods for administering a pharmaceutical composition of the present invention may be accomplished by either oral or parenteral administration. A particularly preferred administration method is parenteral administration. Specifically, this administration method includes, for example, administration by injection, transnasal administration, transpulmonary administration, and transdermal administration. As an example of administration by injection, a pharmaceutical composition of the present invention can be administered systemically or locally by intravenous injection, intramuscular injection, intraperitoneal injection, subcutaneous injection, or such.

The present invention provides methods for suppressing proliferation of a neuromedin U receptor 2 (FM4) protein-expressing cell, which comprise the step of contacting a neuromedin U receptor 2 (FM4) protein-expressing cell with a ligand of the neuromedin U receptor 2 (FM4) protein.

The present invention provides methods for suppressing colony formation, which comprise the step of contacting a neuromedin U receptor 2 (FM4) protein-expressing cell with a ligand of the neuromedin U receptor 2 (FM4) protein.

The present invention provides methods for suppressing cell metastasis, which comprise the step of contacting a neuromedin U receptor 2 (FM4) protein-expressing cell with a ligand of the neuromedin U receptor 2 (FM4) protein.

A ligand for the neuromedin U receptor 2 (FM4) protein is as described for a ligand of the neuromedin U receptor 2 (FM4) protein of the present invention. Cells to which a ligand of the neuromedin U receptor 2 (FM4) protein binds are not particularly limited as long as they are cells expressing the neuromedin U receptor 2 (FM4) protein, but they are preferably cancer cells and more preferably pancreatic cancer cells.

In the present invention, "contact" is carried out, for example, by adding a ligand of the neuromedin U receptor 2 (FM4) protein to a culture medium of neuromedin U receptor 2 (FM4) protein-expressing cells grown in a test tube. In this case, as a carrier of the ligand to be added, a carrier such as a solution or a solid obtained by freeze-drying or such can be suitably used. When the ligand is added as an aqueous solution, the aqueous solution may purely contain only the ligand. Alternatively, the aqueous solution may be a solution containing, for example, surfactants, excipients, coloring agents, flavoring agents, preservatives, stabilizers, buffers, suspending agents, isotonizing agents, binders, disintegrants, lubricants, fluidity accelerators, and corrigents exemplified by light anhydrous silicic acid, lactose, crystalline cellulose, mannitol, starch, carmellose calcium, carmellose sodium, hydroxypropyl cellulose, hydroxypropyl methyl cellulose, polyvinyl acetal diethylaminoacetate, polyvinylpyrrolidone, gelatin, medium-chain triglyceride-fatty acid, polyoxyethylene hardened castor oil 60, sucrose, carboxymethyl cellulose, cornstarch, inorganic salts, and such. The concentration at which the ligand is added is not particularly limited, but a final concentration in the range of preferably 1 pg/mL to 1 g/mL, more preferably 1 ng/mL to 1 mg/mL, and even more preferably 1 µg/mL to 1 mg/mL culture solution may suitably be used.

In another embodiment, "contact" in the present invention can also be carried out by administration to non-human animals that have neuromedin U receptor 2 (FM4) protein-expressing cells transplanted into their bodies, or to animals carrying cancer cells that endogenously express the neuromedin U receptor 2 (FM4) protein. Oral or parenteral administration can be carried out for the administration method. Parenteral administration methods are particularly preferred, and specific examples of this administration method include administration by injection, transnasal administration, transpulmonary administration, and transdermal administration. As an example of administration by injection, a pharmaceutical agent of the present invention (cell proliferation-suppressing agent, agent for suppressing colony formation, agent for suppressing cell movement, cancer therapeutic agent, and agent for suppressing cancer metastasis) may be administered systemically or locally by intravenous injection, intramuscular injection, intraperitoneal injection, subcutaneous injection, or such. A suitable administration method may be selected according to the age and symptoms of the test animal. When administered as an aqueous solution, the aqueous solution may purely contain only the ligand. Alternatively, the aqueous solution may be a solution containing, for example, the above-described surfactants, excipients, coloring agents, flavoring agents, preservatives, stabilizers, buffers, suspending agents, isotonizing agents, binders, disintegrants, lubricants, fluidity accelerators, and corrigents. The dose per administration can be selected, for example, in the range of 0.0001 mg to 1000 mg per kg body weight. Alternatively, the dose can be selected, for example, in the range of 0.001 to 100,000 mg/body per subject. However, the dose of a ligand of the present invention is not limited to such doses.

As a method for evaluating or measuring the cell proliferation-suppressing activity induced in neuromedin U receptor 2 (FM4) protein-expressing cells due to contact with a ligand of the neuromedin U receptor 2 (FM4) protein in a test tube, for example, the method described in the above-mentioned 2(7) can be suitably used. Furthermore, a method for evaluating or measuring the cell proliferation-suppressing activity *in vivo* is, for example, transplanting neuromedin U receptor 2 (FM4) protein-expressing cancer cells intradermally or subcutaneously to a non-human test animal, and then administering a carrier containing a test ligand intravenously or intraperitoneally, every day or in intervals of days from the day of transplantation or from the following day. The cell proliferation-suppressing activity can be defined by successive daily measurement of the size of the tumor. As with the evaluation in a test tube, cell proliferation-suppressing activity can be determined by administering a carrier that does not contain a ligand of the neuromedin U receptor 2 (FM4) protein, and observing whether the size of the tumor in the group which received administration of the ligand of the neuromedin U receptor 2 (FM4) protein is significantly smaller than the size of the tumor in the control group without administration of the ligand. When using mice as the non-human test animal, it is suitable to use a nude (nu/nu) mouse whose thymus has been made genetically defective so that its T lymphocyte function is lost. By using such mice, involvement of T-lymphocytes in the test animals can be eliminated when evaluating and measuring the cell proliferation-suppressing activity due to the administered ligand.

Furthermore, as a method for evaluating or measuring in a test tube, the colony formation-suppressing activity induced in neuromedin U receptor 2 (FM4) protein-expressing cells due to contact with a ligand of the neuromedin U receptor 2 (FM4) protein, the method described in the above-mentioned 2(8), for example, can be suitably used. In addition, as a method for evaluating or measuring in a test tube, the cell movement-suppressing activity induced in neuromedin U receptor 2 (FM4) protein-expressing cells due to contact with a ligand of the neuromedin U receptor 2 (FM4) protein, the method described in the above-mentioned 2(9), for example, can be suitably used.

All prior art references cited herein are incorporated by reference into this description.

### Examples

Herein below, the present invention will be specifically described with reference to the Examples, but it is not to be construed as being limited thereto.

### 1. Expression of NmU-R2 (FM4) in pancreatic cancer

### 1-1. Human NmU gene expression analysis using Gene chip

To search for genes whose expression is enhanced specifically in cancer tissues such as lung cancer or pancreatic cancer tissues, comprehensive gene expression analyses were carried out on normal tissues, cancer tissues, and cancer cell lines using GeneChip U133A (manufactured by Affymetrix).

First, total RNAs were prepared by common procedures from the normal tissues, cancer tissues, and cancer cell lines shown in Tables 1 and 2 using ISOGEN (manufactured by Nippon Gene). Gene expression analyses were carried out according to the Expression Analysis Technique Manual (manufactured by Affymetrix) using 10 µg each of these total RNAs, and subjecting them to GeneChip U-133A (manufactured by Affymetrix). The mean value of the expression score for the total genes was set to 100, and then genes showing enhanced expression in cancer tissues or cancer cells were searched.

**Table 1**

| Tissue | Origin |
|---|---|
| Whole brain | Clontech 64020-1 |
| Lung | Clinical sample, 1 case |
| Trachea | Clontech 64091-1 |
| Heart | Ambion 7966 |
| Kidney | Ambion 7976 |
| Liver | Clinical sample (Surgery) |
| Pancreas | Ambion 7954 |
| Stomach | Clinical sample (Surgery) |
| Small Intestine | Ambion 7984 |
| Large Intestine | Ambion 7986 |
| Bone marrow | Clontech 64106-1 |
| Peripheral mononuclear blood cell | Clinical sample, 1 case |
| Testis | Clontech 64027-1 |
| Prostate | Ambion 7988 |
| Ovary | Ambion 7974 |
| Skin | Stratagene 735031 |
| Small cell lung cancer 1 | Clinical sample, 1 case |
| Small cell lung cancer 2 | Clinical sample, 1 case |
| Small cell lung cancer 3 | Clinical sample, 1 case |
| Small cell lung cancer 4 | Clinical sample, 1 case |
| Small cell lung cancer 5 | Clinical sample, 1 case |
| Small cell lung cancer 6 | Clinical sample, 1 case |
| Small cell lung cancer 7 | Clinical sample, 1 case |
| Small cell lung cancer 8 | Clinical sample, 1 case |
| Small cell lung cancer 9 | Clinical sample, 1 case |
| Small cell lung cancer 10 | Clinical sample, 1 case |
| Lung squamous cell carcinoma 1 | Clinical sample, 1 case |
| Lung squamous cell carcinoma 2 | Clinical sample, 1 case |
| Lung squamous cell carcinoma 3 | Clinical sample, 1 case |
| Lung squamous cell carcinoma 4 | Clinical sample, 1 case |
| Lung squamous cell carcinoma 5 | Clinical sample, 1 case |
| Lung adenocarcinoma 1 | Clinical sample, 1 case |
| Lung adenocarcinoma 2 | Clinical sample, 1 case |
| Lung adenocarcinoma 3 | Clinical sample, 1 case |
| Lung adenocarcinoma 4 | Clinical sample, 1 case |
| Lung adenocarcinoma 5 | Clinical sample, 1 case |
| Pancreatic cancer 1 | Clinical sample, 1 case |
| Pancreatic cancer 2 | Clinical sample, 1 case |
| Pancreatic cancer 3 | Clinical sample, 1 case |
| Pancreatic cancer 4 | Clinical sample, 1 case |

**Table 2**

| Cancer type | Cell line | medium | Serum (%) |
|---|---|---|---|
| Brain tumor | U251 | DMEM | 10 |
| Breast cancer | MCF7 | RPMI1640 | 10 |
| Esophageal cancer | TE2 | RPMI1640 | 10 |
| Stomach cancer | AGS | RPMI1640 | 10 |
| | GT3 | DMEM | 10 |
| | KatoIII | RPMI1640:DMEM=1:1 | 10 |
| | MKN45 | RPMI1640 | 10 |
| | MKN74 | RPMI1640 | 10 |
| | 2M | DMEM | 10 |
| | 2MD3 | DMEM | 10 |
| Colon cancer | CACO2 | DMEM | 20 |
| | DLD1 | RPMI1640 | 10 |
| | hCT 116 | McCoy5A | 10 |
| | LOVO | HamF 12:DMEM=1:1 | 10 |
| | SW480 | RPMI1640 | 10 |
| Liver cancer | Alexander | DMEM | 10 |
| | HepG2 | DMEM | 10 |
| | HLE | DMEM | 10 |
| | HuH6 | DMEM | 10 |
| | HuH7 | DMEM | 10 |
| Pancreatic cancer | Capan1 | DMEM | 20 |
| | KLM1 | RPMI1640 | 10 |
| | Panc1 | RPMI1640 | 10 |
| | PK59 | RPMI1640 | 10 |
| | PK-1 | RPMI1640 | 10 |
| Kidney cancer | Caki1 | RPMI1640 | 10 |
| | Caki2 | RPMI1640 | 10 |
| Lung cancer | A549 | DMEM | 10 |
| | Lu130 | RPMI1640 | 10 |
| | H1359 | RPMI1640 | 10 |
| | H157 | RPMI1640 | 10 |
| | H1648 | HamF12:DMEM=1:1 | 10 |
| | H2009 | HamF12:DMEM=1:1 | 10 |
| | H23 | RPMI1640 | 10 |
| | H2347 | RPMI1640 | 10 |
| | H522 | RPMI1640 | 10 |
| Cervical cancer | Hela | DMEM | 10 |

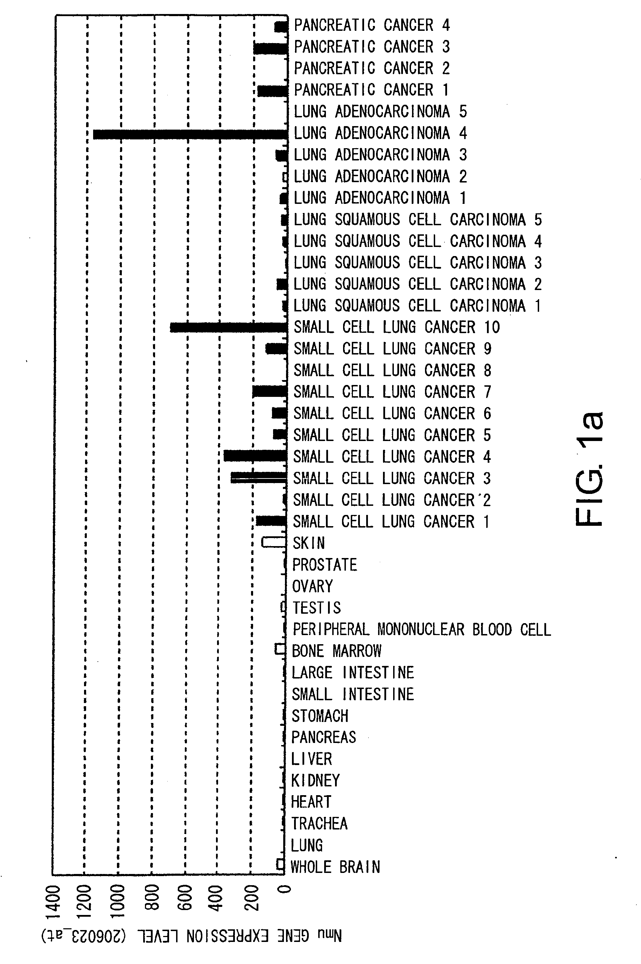

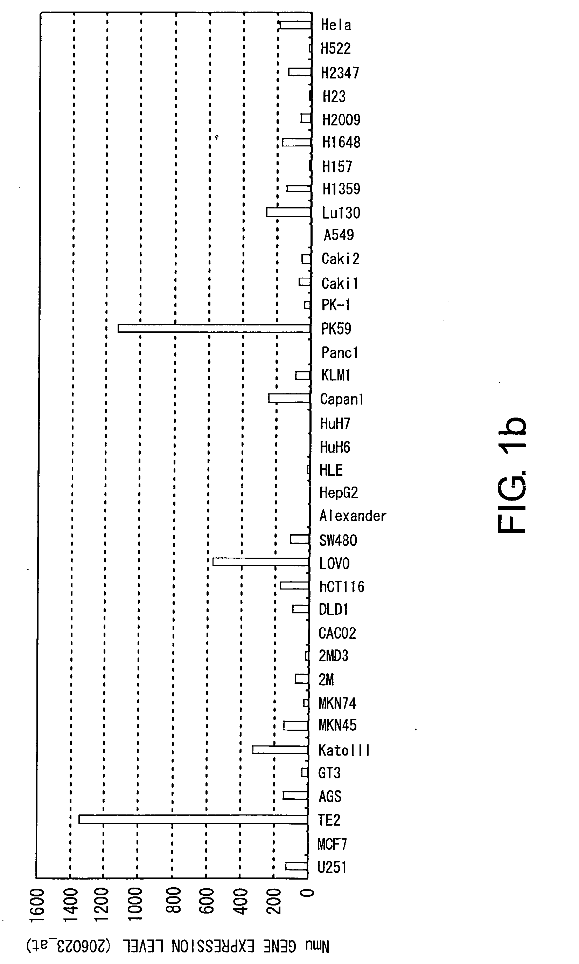

As a result, while the human NmU gene (probe ID: 206023_at HG-U133A) did not show significant expression in the normal tissues examined, its expression was enhanced in lung adenocarcinoma, lung squamous cell carcinoma, small cell lung cancer, and pancreatic cancer tissues. Cancer cell lines in which the NmU gene showed a score of 100 or more were brain tumor (U251), esophageal cancer (TE2), stomach cancer (AGS, KATOIII, MKN45, and 2M), colon cancer (DLD1, hCT116, LOVO, and SW480), pancreatic cancer (Capan1, KLM1, and PK59), lung cancer (Lu130, H1395, H1648, and H2347), and cervical cancer (Hela) cell lines (Figs. 1(A) and (B)).

Accordingly, it was found that while the human NmU gene (probe ID: 206023_at HG-U133A) had very low expression levels in normal tissues, its expression was enhanced in a wide variety of cancer types such as lung cancer, colon cancer, pancreatic cancer, stomach cancer, and kidney cancer.

### 1-2. Expression analyses of NmU and its receptor gene by RT-PCR

Expression of NmU was found to be increased in a number of pancreatic cancer patients by GeneChip analysis. Therefore, with the objective of performing a detailed analysis on the actual gene expression in pancreatic cancer, the expression of NmU and its receptors, FM3 and FM4, in pancreatic cancer was analyzed by RT-PCR.

The specific procedure is as follows. First, 12 types of pancreatic cancer cell lines (BxPC-3, CFPAC-1, PANC-1, HPAC, MIApaca, Capan-1, Capan-2, HPAF II, AsPc, HS766T, Mpanc96, and Su.86.86; all purchased from American Type Culture Collection (ATCC)) were cultured under culturing conditions described in the ATCC Handbook. Each cell was dissolved in Trizol (Invitrogen) and total RNA was prepared from the cell. cDNA was synthesized from this RNA according to the attached manual (SuperScript II First-Strand System; Invitrogen), and the obtained pancreatic cancer cell line-derived cDNA and cDNAs from various normal human organs (human Marathon-Ready cDNA; Clontech) were used as templates to perform RT-PCR using EX Taq polymerase (Takara). The amplification conditions and the primer sets used for each gene are shown below.
NmU (94°C for 30 seconds, 60°C for 30 seconds, 72°C for 30 seconds: 30 cycles)
NmU-1: CTCAGGCATCCAACGCACTG (SEQ ID NO: 1)
NmU-2: CTGACCTTCTTCCATTCCGTG (SEQ ID NO: 2)
FM3 (94°C for 30 seconds, 60°C for 30 seconds, 72°C for 30 seconds: 34 cycles)
NmUR1-1: GCTATTTCCGCACGCTACTGT (SEQ ID NO: 3)
NmUR1-2: GCCCAATGAGCAGGTAGAGC (SEQ ID NO: 4)
FM4 (94°C for 30 seconds, 60°C for 30 seconds, 72°C for 30 second: 34 cycles)
NmUR2-1: GGGCTGCTACTTCAAGACGG (SEQ ID NO: 5)
NmUR2-2: CCCTTCATCTGCCTCAAGAGA (SEQ ID NO: 6)

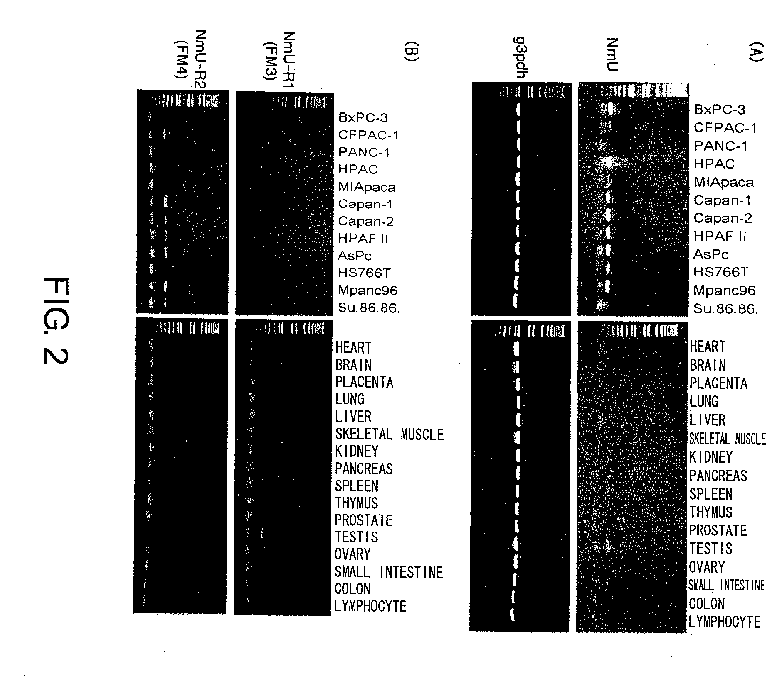

As a result of the RT-PCR analyses, the NmU expression was observed in ten out of twelve cell lines (Fig. 2(A)). While expression of one of the receptors, FM4, could not be observed at all in normal peripheral tissues, its expression was confirmed at a high frequency in seven out of twelve pancreatic cancer cell lines. The other receptor, FM3, was observed to be expressed in peripheral tissues such as the liver, pancreas, spleen, testis, and small intestine, but its expression was not observed at all in pancreatic cancer cell lines (Fig. 2 (B)).

The finding that the FM4 gene is enhanced in pancreatic cancer as described is a novel finding, and this strongly suggested that FM4 may be useful as a target molecule for molecule-targeting therapeutic agents against pancreatic cancer.

### 2. Proliferation-suppressing effect of NmU mediated by NmU-R2 (FM4)

### 2-1. Construction of FM4 expression vector

To construct an FM4 expression vector, first, the FM4 gene was cloned as follows. Pancreatic cancer cell line (Capan-1)-derived cDNAs were used as templates for RT-PCR performed under the following conditions using Pyrobest Taq polymerase (Takara) to clone the full-length FM4 gene.
FM4-UP: ATGTCAGGGATGGAAAAACTTC (SEQ ID NO: 7)
FM4-LOW: TCAGGTTTTGTTAAAGTGGAAGC (SEQ ID NO: 8)
(94°C for 30 seconds, 59°C for 30 seconds, 72°C for 60 seconds: 32 cycles)

Next, the obtained PCR products were used as templates for another round of PCR under the following conditions to obtain a full-length FM4 cDNA fragment with EcoRI and NotI cleavage sequences added to its 5' end and 3' end, respectively.
FM4-ECO: AAAGAATTCCACCATGTCAGGGATGGAAAAACTTCAGAA (SEQ ID NO: 9)
FM4-NOT: TTTGCGGCCGCTCAGGTTTTGTTAAAGTGGAAGCTTT (SEQ ID NO: 10)
(94°C for 30 seconds, 68°C for 30 seconds, 72°C for 60 seconds: 20 cycles)

These were cleaved with EcoRI and NotI, and then inserted into an animal expression vector (pMCN) that had been similarly cleaved with EcoRI and NotI to construct the FM4 expression vector (pMCN-FM4).

### 2-2. Establishment of FM4-expressing CHO cell line

15 µg of linearized FM4 expression vector (pMCN-FM4) obtained by digestion with PvuI was introduced into CHO cells by electroporation (Gene Pulser; BioRad) at 1.5 kV, 25 µFD. The cells were cultured in a medium containing 500 µg/mL of G418, and G418-resistant cells were picked. NmU was added to these cells, and cell lines that reacted to NmU were selected using the increase in intracellular Ca²⁺ concentration as an index.

### 2-3. Measurement of intracellular calcium concentration

The cells were detached using trypsin, plated onto a 96-well plate (black plate for fluorescence measurement) at 2 x 10⁴ cells/well, and after culturing overnight, they were used for intracellular calcium measurements. Measurement of the intracellular calcium concentration was performed as follows.

After removing the medium, an Fluo 4 AM solution [2% FCS, 3 µM Fluo 4 AM (Molecular PROBES) in assay buffer (2 mM HEPES, 1.5 mM probenecid in HBSS)] was added at 50 µL/well. After incubation in the dark at 37°C for 30 minutes, the cells were washed three times in the assay buffer. Then, an assay buffer that contains 2.5 µM of NmU (Funakoshi) was added, and the intracellular Ca²⁺-dependent fluorescence trace at 490-nm excitation was monitored on a plate reader for fluorimetric analysis (Fusion; Perkin Elmer).

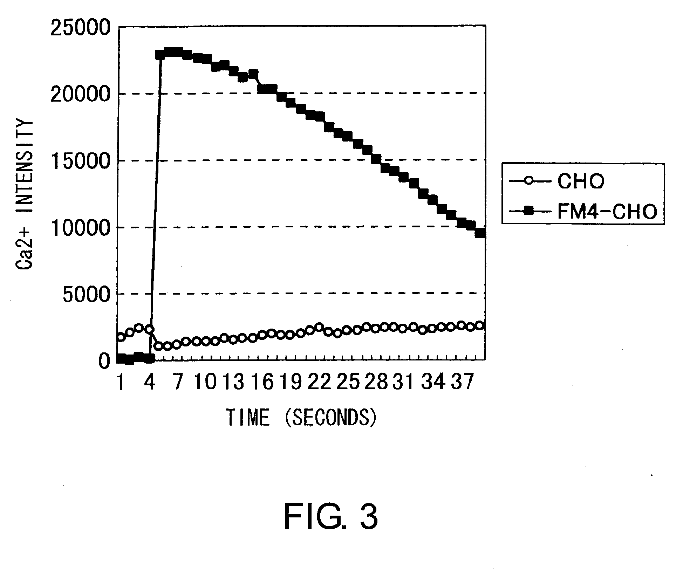

The intracellular Ca²⁺ concentration of the established FM4-CHO cell line quickly increased as a result of NmU stimulation (Fig. 3). This confirmed that in the obtained cells, the NmU signaling is transmitted intracellularly via FM4.

### 2-4. Analysis of proliferation-suppressing effect ofNmU

FM4-mediated NmU signaling was analyzed using an FM4-expressing CHO cell line (Fm4-CHO) which was screened using the increase in intracellular Ca²⁺ concentration by NmU stimulation as an index.

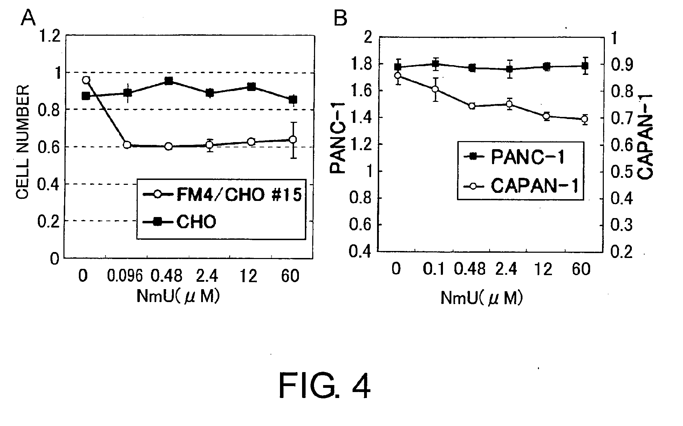

FM4-CHO cells were plated onto a 96-well plate at 2 x 10³ cells/well. On the following day, NmU was added to each well at various concentrations (0.096 µM to 60 µM) and the cells were cultured. 48 Hours later, the number of viable cells were analyzed by WST-8 assay (Cell counting kit-8, Dojindo Laboratories). The results showed that NmU suppressed proliferation of FM4-CHO at low concentration (Fig. 4A).

Next, whether NmU also shows proliferation-suppressing effects in a pancreatic cancer cell line that highly expresses FM4 was examined. Capan-1, which shows high FM4 gene expression in RT-PCR analysis, and PANC-1, from which the FM4 gene expression was detected, were selected and these cells were plated onto 96-well plates. On the following day, NmU was added to each well at 0.096 µM to 60 µM. Seventy-two hours later, the number of cells was determined. The results showed that, while NmU did not act at all on the proliferation of PANC-1, the proliferation-suppressing effect of NmU was observed in Capan-1 which has high FM4 gene expression (Fig. 4B).

### 3. NmU-R2 (FM4)-mediated suppressive effect of NmU on colony formation

### 3-1. Establishment of an FM4-expressing CHO cell line

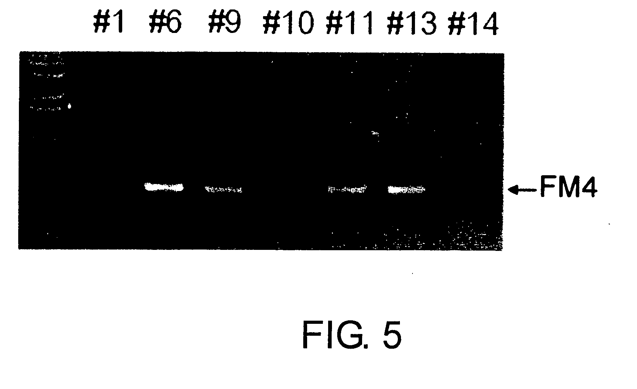

A cell line with forced expression of FM4 was established as follows for the pancreatic cancer cell line PANC-1. 15 µg of the linearized FM4 expression vector (pMCN-FM4) obtained by PvuI digestion was introduced into CHO cells by electroporation (Gene Pulser; BioRad) at 1.5 kV, 25 µFD. The cells were cultured in a medium containing 400 µg/mL of G418, and seven clones of G418-resistant cells were picked. Total RNAs were purified from these cells and cDNAs were synthesized. The obtained cDNAs were used as template and FM4 expression was analyzed by RT-PCR (Fig. 5). Since Clone #6 showed the highest expression of the FM4 gene, this cell line was used in the following experiments as FM4-expressing PANC-1 (FM4-PANC1).

### 3-2. Colony formation-suppressive effect of NmU on FM4-expressing PANC1 cells

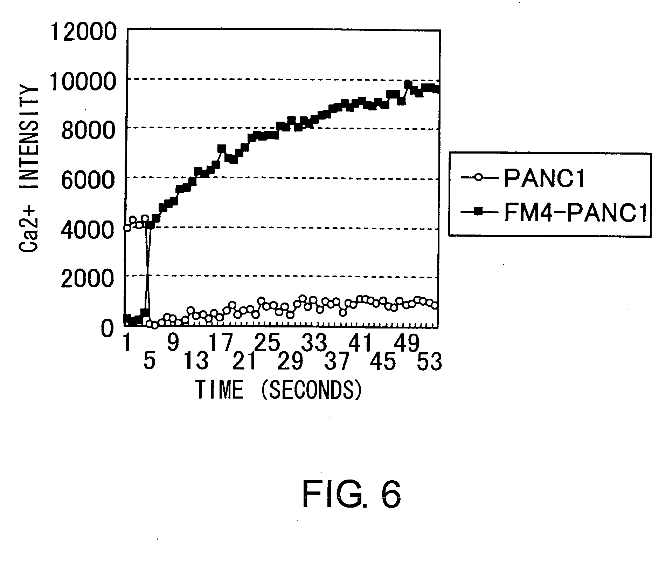

In order to confirm that the NmU signaling is transmitted into the cell in the obtained FM4-expressing PANC1 cells (FM4-PANC1), whether the addition of NmU increased the intracellular Ca²⁺ concentration was analyzed. As a result, increase of the intracellular Ca²⁺ concentration due to addition of NmU (1 µM) was confirmed in FM4-PANC1 (Fig. 6). This confirmed that in the established FM4-PANC1 cells, NmU stimulation causes the FM4-mediated signaling to be transmitted into the cell.

To analyze the effect of NmU on the ability of this cell to form colonies, soft agar colony formation assay was performed. Soft agar colony formation assay was performed as follows.

Base agar (0.5% agar, 1x MEM, and 10% FCS) was added to each well of a 6-well plate at 1.5 mL/well, and then 1.5 mL/well (0.5 x 10⁴ cells/well) of PANC 1 cells or FM4-PANC1 cells in the presence or absence of NmU (1 µM) in top agar (0.35% agar, 1x MEM, and 10% FCS) was added at to each of the wells. After culturing for one month, the number of colony forming cells in the soft agar was counted under a microscope.

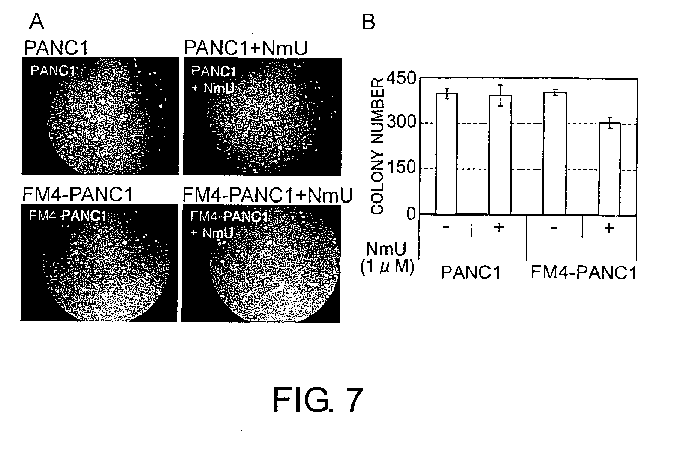

As a result, NmU did not have any effect on the colony forming ability of the parent cell line that does not express FM4; however, the colony formation-suppressing effect by NmU was confirmed in FM4-PANC1 (Fig. 7).

The above-mentioned results showed that NmU suppresses colony formation in a soft agar culture through FM4.

### 4. NmU-R2 (FM4)-mediated suppressive effect of NmU on cell movement

### 4-1. Alteration of cell morphology in FM4-CHO cells

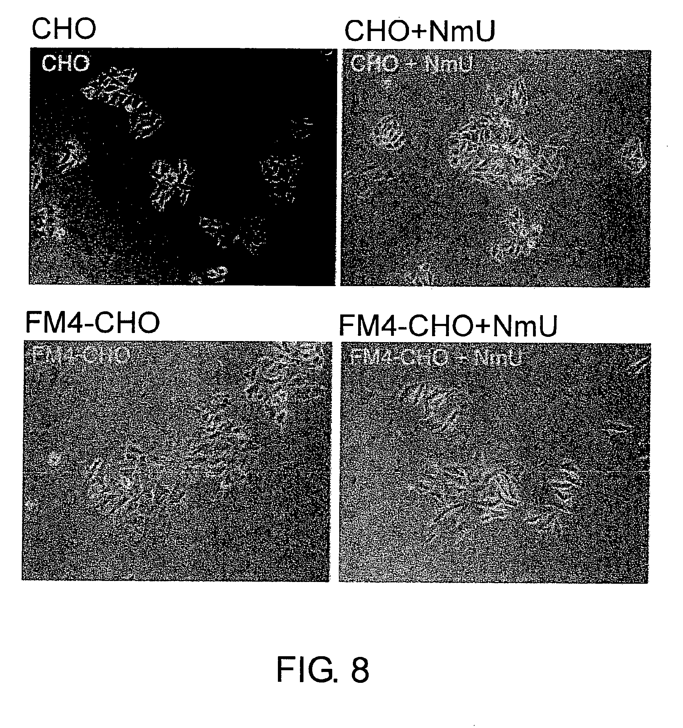

The cell morphology of FM4-CHO cells which were forced to express FM4 was observed under a microscope. As a result, morphological alterations of the cells were observed in characteristics such as increased cell protrusion and cell enlargement in FM4-CHO cells as compared to CHO cells which are the parent cell line (Fig. 8).

When FM4-CHO cells having such characteristics were further stimulated with NmU, significant morphological alterations of the cells such as disappearance of cell protrusions were observed in approximately 12 hours or so (Fig. 8). This led to the speculation that NmU is involved in signal transduction that regulates cell adhesion and cell movement.

### 4-2. Suppressive effect of NmU on cell movement ability

Next, the effect of NmU on cell movement ability was analyzed by wound healing assay. Parent cell line CHO cells and FM4-CHO cells were grown to confluency in plates, and wounded with a pipette tip to make a fixed clearance between the cells. After washing the cells twice with PBS, the cells were cultured in a serum-free culture medium in the presence or absence of NmU (5 µM). Twenty-four hours later, cells that moved into the clearance between the cells were observed and photographed under a microscope.

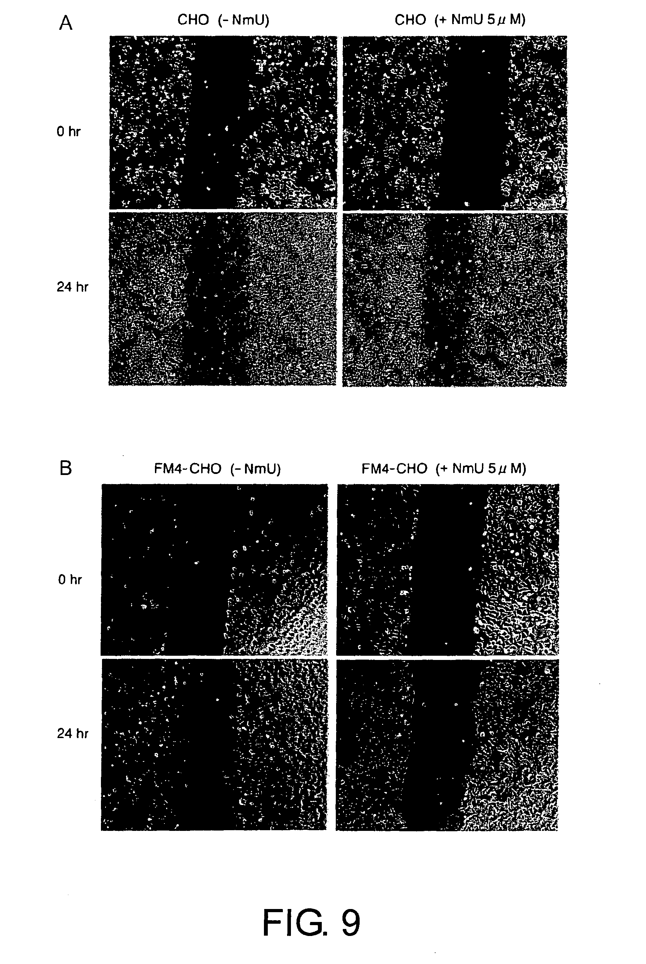

As a result, NmU did not have any effect on cell movement of the parent CHO cells, but in contrast, the cell movement of FM4-CHO cells was completely inhibited by the NmU stimulation (Fig. 9). This strongly suggested that NmU has the effect of suppressing cell movement through FM4.

### Industrial Applicability

Ligands for the neuromedin U receptor 2 (FM4) molecule of the present invention can be used as cell proliferation-suppressing agents, agents for suppressing colony formation, or agents for suppressing cell movement for various types of cancer cells, such as pancreatic cancer cells, that express the neuromedin U receptor 2 (FM4) molecule. Furthermore, ligands for the neuromedin U receptor 2 (FM4) molecule of the present invention can be used as cancer therapeutic agents against cancers such as pancreatic cancer. In addition, they can be used as post-operative prophylactic agents for cancers such as pancreatic cancer.

Furthermore, neuromedin U receptor 2 (FM4) molecules of the present invention can be used as diagnostic markers for cancers such as pancreatic cancer. More specifically, by using a probe that can detect a neuromedin U receptor 2 (FM4) molecule after labeling it with a chemical substance or a radioisotope, the presence of pancreatic cancer can be detected *ex vivo* or *in vivo.*

## Claims

1. A cancer therapeutic agent comprising as an active ingredient a ligand for a polypeptide comprising the amino acid sequence of SEQ ID NO: 12 or a polypeptide functionally equivalent thereto.

2. The cancer therapeutic agent of claim 1, wherein the ligand is a polypeptide comprising the amino acid sequence of SEQ ID NO: 14 or a polypeptide functionally equivalent thereto.

3. The cancer therapeutic agent of claim 1 or 2, wherein the cancer is pancreatic cancer.

4. A cancer metastasis-suppressing agent comprising as an active ingredient a ligand for a polypeptide comprising the amino acid sequence of SEQ ID NO: 12 or a polypeptide functionally equivalent thereto.

5. The cancer metastasis-suppressing agent of claim 4, wherein the ligand is a polypeptide comprising the amino acid sequence of SEQ ID NO: 14 or a polypeptide functionally equivalent thereto.

6. The cancer metastasis-suppressing agent of claim 4 or 5, wherein the cancer is pancreatic cancer.

7. A cell proliferation-suppressing agent comprising as an active ingredient a ligand for a polypeptide comprising the amino acid sequence of SEQ ID NO: 12 or a polypeptide functionally equivalent thereto.

8. The cell proliferation-suppressing agent of claim 7, wherein the ligand is a polypeptide comprising the amino acid sequence of SEQ ID NO: 14 or a polypeptide functionally equivalent thereto.

9. The cell proliferation-suppressing agent of claim 7 or 8, wherein the cells are pancreatic cancer cells.

10. A method of screening for a ligand for a polypeptide comprising the amino acid sequence of SEQ ID NO: 12 or a polypeptide functionally equivalent thereto, which comprises the steps of:
(a) contacting a test substance with a cell expressing a polypeptide comprising the amino acid sequence of SEQ ID NO: 12 or a polypeptide functionally equivalent thereto, or with an extract of said cell;
(b) measuring cell-stimulating activity of the test substance in the cell of step (a) or the extract of said cell; and
(c) selecting a test substance that alters the above-mentioned cell-stimulating activity as compared to when the test substance is not contacted.

11. A method of screening for a ligand for a polypeptide comprising the amino acid sequence of SEQ ID NO: 12 or a polypeptide functionally equivalent thereto, which comprises the steps of:
(a) contacting a test substance with a cell expressing a polypeptide comprising the amino acid sequence of SEQ ID NO: 12 or a polypeptide functionally equivalent thereto, or with an extract of said cell;
(b) measuring cell-stimulating activity of the test substance in the cell of step (a) or the extract of said cell; and
(c) selecting a test substance that alters the above-mentioned cell-stimulating activity as compared to when neuromedin U is contacted.

12. The method of claim 10 or 11, wherein the cell is a recombinant cell.

13. The method of claim 12, wherein the recombinant cell is a cell derived from CHO or PANC1.

14. The method of any one of claims 10 to 13, wherein the cell-stimulating activity is intracellular Ca²⁺ concentration-increasing activity.

15. The method of any one of claims 10 to 13, wherein the cell-stimulating activity is cell proliferation-suppressing activity.

16. The method of any one of claims 10 to 13, wherein the cell-stimulating activity is activity of suppressing cell colony formation.

17. The method of any one of claims 10 to 13, wherein the cell-stimulating activity is activity of suppressing cell movement.

18. A ligand obtained by the method of any one of claims 10 to 17.

19. A method for diagnosing a cancer, which comprises the step of detecting the expression level of a polynucleotide encoding a polypeptide comprising the amino acid sequence of SEQ ID NO: 12 or a polypeptide functionally equivalent thereto in a biological sample of a specimen,
wherein the specimen is diagnosed with cancer when the expression level of a polynucleotide encoding the polypeptide comprising the amino acid sequence of SEQ ID NO: 12 or the polypeptide functionally equivalent thereto is increased as compared to a normal tissue.

20. The method of claim 19, wherein the detection is performed using as a probe a polynucleotide encoding a polypeptide comprising the amino acid sequence of SEQ ID NO: 14 or a polypeptide functionally equivalent thereto, or a fragment thereof.

21. The method of claim 20, wherein the polynucleotide comprises the nucleotide sequence of SEQ ID NO: 13.

22. The method of any one of claims 19 to 21, wherein the cancer is pancreatic cancer.

23. A method for treating cancer, which comprises the step of administering to a subj ect a ligand for a polypeptide comprising the amino acid sequence of SEQ ID NO: 12 or a polypeptide functionally equivalent thereto.

24. A method for suppressing cancer metastasis, which comprises the step of administering to a subject a ligand for a polypeptide comprising the amino acid sequence of SEQ ID NO: 12 or a polypeptide functionally equivalent thereto.

25. A method for suppressing cell proliferation, which comprises the step of administering to a subject a ligand for a polypeptide comprising the amino acid sequence of SEQ ID NO: 12 or a polypeptide functionally equivalent thereto.

26. Use of a ligand for a polypeptide comprising the amino acid sequence of SEQ ID NO: 12 or a polypeptide functionally equivalent thereto in the manufacture of a cancer therapeutic agent.

27. Use of a ligand for a polypeptide comprising the amino acid sequence of SEQ ID NO: 12 or a polypeptide functionally equivalent thereto in the manufacture of a cancer metastasis-suppressing agent.

28. Use of a ligand for a polypeptide comprising the amino acid sequence of SEQ ID NO: 12 or a polypeptide functionally equivalent thereto in the manufacture of a cell proliferation-suppressing agent.
